(19) Europäisches Patentamt
European Patent Office
Office européen des brevets

(11) **EP 2 533 741 B1**

(12) **EUROPÄISCHE PATENTSCHRIFT**

(45) Veröffentlichungstag und Bekanntmachung des Hinweises auf die Patenterteilung:
**09.10.2013 Patentblatt 2013/41**

(21) Anmeldenummer: **11703142.7**

(22) Anmeldetag: **02.02.2011**

(51) Int Cl.:
***A61F 13/15*** *(2006.01)*

(86) Internationale Anmeldenummer:
**PCT/EP2011/000466**

(87) Internationale Veröffentlichungsnummer:
**WO 2011/098226 (18.08.2011 Gazette 2011/33)**

(54) **INKONTINENZARTIKEL IN HÖSCHENFORM**

INCONTINENCE ARTICLE IN THE FORM OF PANTIES

ARTICLE POUR INCONTINENCE EN FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.02.2010 DE 102010007872**

(43) Veröffentlichungstag der Anmeldung:
**19.12.2012 Patentblatt 2012/51**

(73) Patentinhaber: **Paul Hartmann AG**
**89522 Heidenheim (DE)**

(72) Erfinder: **OSTERTAG, Wolfgang**
**89547 Gerstetten (DE)**

(56) Entgegenhaltungen:
**EP-A1- 0 172 037    EP-A1- 0 405 575**
**EP-A2- 1 327 430    WO-A1-99/37480**
**US-A- 4 492 608    US-A- 4 795 451**
**US-A- 5 507 909    US-A1- 2001 030 014**

EP 2 533 741 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt und Rückenabschnitt erstreckt und an den Bauchabschnitt und an den Rückenabschnitt unlösbar angefügt ist, wobei sowohl der Schrittabschnitt als auch der Bauchabschnitt und der Rückenabschnitt die Beinöffnungen des Inkontinenzartikels begrenzen. Ein derart dreikomponentiger Inkontinenzartikel ist beispielsweise aus WO 2004/052260 A1, WO 03/039423 A1 und WO-2009/065499- A1 bekannt. Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person. Inkontinenzartikel in Höschenform unterscheiden sich prinzipiell von traditionellen öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra- Fäden bezeichnet, in vorgedehntem Zustand (Stretch- Bond- Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der inkontinenzartikel wie ein Höschen an den Benutzer angelegt wird. Inkontinenzartikel in Höschenform mit derart elastifizierten Chassismaterialien sind mehrfach bekannt geworden und beispielsweise auch in den vorerwähnten WO 2004/052260 A1 und WO- 2009/065499- A1 behandelt.

Es wurde mit der vorliegenden Erfindung erkannt, dass beim Anlegevorgang des Inkontinenzartikels in Höschenform und zwar beim Aufweiten der durch die Vorspannung der Elastifizierungsmittel zusammengerafften Chassismaterialien es dennoch zu nicht unerheblichen Krafteinwirkungen kommen kann. Unter anderem abhängig vom Vorgang des Anlegens durch den einzelnen Anwender, wirken diese Kräfte aus unterschiedlichen Richtungen. So können die Kräfte parallel und/oder in einem Winkel zum Verlauf des Elastifizierungsmittels und damit einhergehend auf die gesamte Laminatstruktur aus Elastifizierungsmittel und Chassismaterial einwirken. Der Inkontinenzartikel in Höschenform wird vor allem im Bereich der Hüftöffnung durch das Auseinanderziehen in Quer- oder Hüftumfangsrichtung durch den Anwender, so zum Erkennen der Beinöffnungen für den Einstieg in den Inkontinenzartikel, aber dann auch durch das Eingreifen in das Chassismaterial für und beim Hochziehen des Inkontinenzartikels entlang des Körpers des Anwenders Krafteinwirkungen ausgesetzt. Dies kann nachteiligerweise zu einer Beeinträchtigung des Zusammenhalts zwischen Elastifizierungsmittel und Chassismaterial bis hin zu einer partiellen Delamination führen, so dass damit die flächenhafte elastifizierende Wirkung der Chassismaterialien gestört und damit die Passform des Inkontinenzartikels in Höschenform negativ beeinflusst wird.

Ebenso wurde mit der vorliegenden Erfindung erkannt, dass die Fixierung der an einem Chassismaterial bereitgestellten Elastifizierungsmittel den Funktionen und den Aufgaben des elastifizierten Bereichs angepasst sein sollte, um so einen hohen Tragekomfort für den Anwender zu gewährleisten.

Eine verfahrensübliche Maßnahme zur Fixierung von Elastifizierungsmittel auf Chassismaterialien ist der Einsatz von Klebemitteln. Es sind verschiedene Methoden zur adhäsiven Fixierung von Elastifizierungsmitteln bekannt. So beschreibt die EP 1 938 777 A2 das vollflächige Beleimen zumindest einer der für eine Sandwich-Positionierung von Elastifizierungsmittel vorgesehenen Vliesbahnen und deren Zusammenführen unter Einlaminierung eines elastischen Materials. Nachteiligerweise führen vollflächige Beleimungen zu einer weitläufigen Versteifung und einer Reduktion der Atmungsaktivität von Materialien, insbesondere von an sich weichen und atmungsaktiven Vliesmaterialien, welche üblicherweise für Inkontinenzartikel eingesetzt werden.

Weiterhin ist bekannt, eine Einzelstrangbeleimung durchzuführen, derart, dass das einzelne Elastifizierungsmittel zuerst mit Kleber umhüllt wird und danach der Lamination zwischen zwei Materialbahnen zugeführt wird. So wird in der WO 2003/039422 A1 das einzelne Elastifizierungsmittel durch eine kammähnliche Nutform eines Kleberauftragkopfes durchgeführt. Zwar wird dadurch der Problematik einer übermäßigen Versteifung und einer übermäßigen Verminderung der Atmungsaktivität der Chassismaterialien entgegengetreten, nachteilig hierbei ist dennoch, dass die Verbindungspunkte zum Erhalt eines Laminats aus Elastifizierungsmittel und Chassismaterial nur am Umfang des Elastifizierungsmittels erreicht werden. Ein ausreichender Zusammenhalt des Laminats kann damit nur durch eine genügend große Anzahl an Elastifizierungsmittel pro Materialabschnitt erreicht werden. Ebenso nachteilig kann hierbei sein, dass durch den direkten Auftrag des Klebers auf das Elastifizierungsmittel die Gefahr der Schädigung des Elastifizierungsmittels zunimmt. Zudem ist es bei einer Einzelstrangbeleimung schwierg, die Menge an kleber, welche auf ein Elastifizierungsmittel, insbesondere auf ein fadenförmiges Elastifizierungsmittel transferiert werden soll, einzustellen. Des Weiteren schlägt auch EP-1830770-A1 die Fixierung von in Querrichtung angeordneten Elastikfäden mittels Einzelstrangbeleimung vor. Auch die WO 2009/080180 A1 sieht vor, die in Querrichtung parallel verlaufenden Elastifizierungsmittel einzeln mit Kleber zu belegen.

Hiervon ausgehend liegt der vorliegenden Erfindung die Aufgabe zugrunde, den vorstehend geschilderten Problemen zu begegnen, also insbesondere eine stabile Verbindung zwischen den den Bauchabschnitt bzw. den Rückenabschnitt bildenden Chassismaterialien und den daran festzulegenden fadenförmigen Elastifizierungsmittel bei einem gattungsgemäßen Inkontinenzartikel in Höschenform auszubilden, ohne dass dies mit einer Beeinträchtigung des Tragekomforts oder weiteren die Funktionalität des Inkontinenzartikels beeinträchtigenden Konsequenzen verbunden ist.

Diese Aufgabe wird nach der Erfindung gelöst durch einen Inkontinenzartikel mit den Merkmalen des Anspruchs 1.

Vorteilhafte Weiterbildungen des Inkontinenzartikels ergeben sich aus den jeweiligen Unteransprüchen.

Die Lösung der Aufgabe besteht bei einem Inkontinenzartikel mit einem dreikomponentigen Aufbau in der erfindungsgemäßen Anordnung der Elastifizierungsmittel und der Kleberzonen:

Der Inkontinenzartikel in Höschenform für die Aufnahme von Körperausscheidungen verfügt über einen vorderen Bauchabschnitt und einen hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind.

Der Inkontinenzartikel verfügt auch über einen einen Absorptionskörper aufweisenden Schrittabschnitt.

Der Schrittabschnitt umfasst ein flüssigkeitsundurchlässiges Backsheet-Material und ein Deckblatt-Material, wobei der Absorptionskörper zwischen Backsheet-Material und Deekblatt-Material angeordnet ist. Das Deckblatt-Material und/oder das Backsheet-Material bilden in Querrichtung einen sich jeweils außerhalb von den Längsrändern des Absorptionskörpers erstreckenden Überhang.

Der Schrittabschnitt mit einem bauchseitigen Längsende und einem rückenseitigen Längsende erstreckt sich in einer Längsrichtung zwischen dem Bauchabschnitt mit einem schrittzugewandten Querrand und dem Rückenabschnitt mit einem schrittzugewandten Querrand.

Dabei überlappt der Schrittabschnitt in einem vorderen Überlappungsbereich den Bauchabschnitt und ist an den Bauchabschnitt unlösbar angefügt, und der Schrittabschnitt überlappt in einem hinteren Überlappungsbereich den Rückenabschnitt und ist an den Rückenabschnitt unlösbar angefügt.

Sowohl der Schrittabschnitt als auch der Bauchabschnitt und der Rückenabschnitt begrenzen die Beinöffnungen des Inkontinenzartikels.

Dabei erstreckt sich das bauchseitige Längsende des Schrittabschnitts in Längsrichtung bis zu einer parallel zur Querrichtung verlaufenden gedachten Linie L1, und das rückenseitige Längsende des Schrittabschnitts erstreckt sich in Längsrichtung bis zu einer parallel zur Querrichtung verlaufenden gedachten Linie L2.

Die Linie L1 teilt den Bauchabschnitt in einen ersten beinöffnungsfernen Bauchteilabschnitt und einen zweiten beinöffnungsnahen Bauchteilabschnitt und die Linie L2 teilt den Rückenabschnitt in einen ersten beinöffnungsfernen Rückenteilabschnitt und einen zweiten beinöffnungsnahen Rückenteilabschnitt.

In dem ersten Bauchteilabschnitt und dem ersten Rückenteilabschnitt sind erste fadenförmige oder bandförmige Elastifizierungsmittel mit einem Fadendurchmesser D vorgesehen, die sich in einem Abstand N voneinander und parallel zueinander in Quer- oder Hüftumfangsrichtung erstrecken und so den ersten Bauchteilabschnitt und den ersten Rückenteilabschnitt flächenhaft elastifizieren.

Der erste Bauchteilabschnitt und der erste Rückenteilabschnitt weisen jeweils mindestens eine erste Lage und eine zweite Lage auf, zwischen denen die ersten Elastifizierungsmittel mittels streifenförmiger Kleberzonen festgelegt sind.

Dabei sind die streifenförmigen Kleberzonen in Querrichtung- oder Hüftumfangsrichtung mit einer Breite G parallel und in einem Abstand M zueinander angeordnet. Dabei ist die Breite G der streifenförmigen Kleberzonen größer als der Fadendurchmesser D der ersten Elastifizierungsmittel und es ist jeweils ein einziges erstes Elastifizierungsmittel innerhalb einer jeweiligen streifenförmigen Kleberzone angeordnet.

Durch die parallele und beabstandete Anordnung von streifenförmigen Kleberzonen werden stabile Laminatstrukturen von ersten faden- oder bandförmigen Elastifizierungsmitteln mit den Chassismaterialien, also einer ersten und einer zweiten Lage, zwischen denen die ersten Elastifizierungsmittel angeordnet sind, gebildet. Dadurch, dass die Breite der streifenförmigen Kleberzone größer ist als der Fadendurchmesser des innerhalb der streifenförmigen Kleberzone jeweils nur einzeln abgelegten fadenförmigen oder bandförmigen ersten Elastifizierungsmittels, wird zum einen eine sichere Fixierung des ersten Elastifizierungsmittels und zum anderen auch eine direkte Fixierung der beiden Lagen miteinander, nämlich durch den verbleibenden nicht durch das erste Elastifizierungsmittel abgedeckten Bereich der Kleberzone erreicht. Dies ist vorteilhaft gegenüber einer Einzelstrangfadenbeleimung, welche eine Verbindung von Materiallage und Elastifizierungsmittel nur entlang des Umfangs des Elastifizierungsmittels ermöglicht. Durch die Abstände der streifenförmigen Kleberzonen wird trotz einer sicheren Laminierung von ersten Elastifizierungsmitteln mit den beiden an das erste Elastifizierungsmittel angrenzenden und dieses einbettenden Lagen der Tragekomfort für den Anwender beibehalten bzw. eingestellt. Zum einen wird die Atmungsaktivität der Chassismaterialien nur in einem geringeren Maße als bei einer vollflächigen Beleimung beeinträchtigt. Zum anderen kann durch die Wahl der Breite und der Abstände der streifenförmigen Kleberzonen die für den Inkontinenzartikel in Höschenform notwendige Stabilität und der vom Anwender gewünschte Tragekomfort eingestellt werden. In Inkontinenzartikeln werden fachüblicherweise nämlich sehr dünne Materialien, wie beispielsweise dünne Vliesmaterialien eingesetzt, die zumeist inhärent auch Eigenschaften, wie hohe Weichheit, hohe Flexibilität und eine hohe Atmungsaktivität aufweisen. Das führt jedoch zu einer vom Nutzer als negativ empfundenen starken Inhomogenität des Rücken- und Bauchabschnittes hinsichtlich der oben beschriebenen Eigenschaften, wie nachfolgend erläutert wird: Ein großer Überhang des Deckblatt-Materials und/oder des Backsheet-Materials in Querrichtung jeweils außerhalb der Längsränder des Absorptionskörpers, und gegebenenfalls außerdem in Längsrichtung außerhalb der Querränder des Absorptionskörpers bringt zwar den Vorteil mit sich, dass aufgrund des großen Überlappungsbereichs von Schrittabschnitt und Bauchabschnitt bzw. von Schrittabschnitt und Rückenabschnitt der Schrittabschnitt herstellerseitig sicher an Rücken- und Bauchabschnitt, insbesondere mittels Klebstoffen festlegbar ist. Es wurde aber erkannt, dass der Überlappungsbereich aufgrund der Vielzahl an Lagen gegenüber dem Bereich außerhalb der Kontur des Schrittabschnitts übermäßig stark versteift ist. Diese starke und konstruktionsbedingte an den Längsrändern und gegebenenfalls an den Querrändern des Schrittabschnitts auftretende abrupte, unvermittelte Eigenschaftsänderung des Rücken- und Bauchabschnitts wird vom Nutzer als unvorteilhaft empfunden, weil der Inkontinenzartikel sich hierdurch signifikant von normaler Unterwäsche unterscheidet. Durch das Festlegen der ersten Elastifizierungsmittel mittels streifenförmiger Kleberzonen in der in Anspruch 1 beschriebenen Weise kann der Bereich des Rücken- und Bauchabschnitts außerhalb der Kontur des Schrittabschnitts gezielt derart mäßig versteift werden, dass dem Nutzer die an den Längsrändern und gegebenenfalls an den Querrändern des Schrittabschnitts auftretende abrupte, unvermittelte Eigenschaftsänderung weniger stark erscheint und der Inkontinenzartikel diesbezüglich die Anmutung eines normalen Unterwäsche-Slips vermittelt.

Mit den Merkmalen des Patentanspruchs 1 wird insgesamt also ein Inkontinenzartikel in Höschenform mit dem genannten dreikomponentigen Aufbau geschaffen, bei dem sich eine sichere Verbindung der Komponenten realisieren lässt, ohne dass dies mit einer Beeinträchtigung des Tragekomforts oder der Funktionalität des Inkontinenzartikels oder seiner Komponenten verbunden wäre, sondern vielmehr zu einer Verbesserung des Tragekomforts führt.

Eine weitere Aufgabe der vorliegenden Erfindung ist die Bereitstellung eines Verfahrens zum Herstellen eines Inkontinenzartikels in Höschenform mit den eingangs genannten Merkmalen, welches insbesondere den vorstehend genannten Aspekten, also insbesondere einer stabilen Verbindung zwischen den den Bauchabschnitt bzw. den Rückenabschnitt bildenden Chassismaterialien und den daran festzulegenden faden- oder bandförmigen Elastifizierungsmittel unter Beibehaltung des Tragekomforts und der Funktionalität Rechnung trägt.

Diese Aufgabe wird gelöst durch ein Verfahren zur Herstellung eines derartigen Inkontinenzartikels mit den Verfahrensschritten gemäß Anspruch 14.

Die absoluten Angaben zu Längen und/oder Breiten des Inkontinenzartikels als solchen bzw. dessen Komponenten, wie beispielsweise Bauchabschnitt, Rückenabschnitt und Schrittabschnitt und auch der streifenförmigen Kleberzonen bzw. Kleberzonenabschnitte sind stets auf Abmessungen am Inkontinenzartikel in seinem flach ausgelegten, gespannten und eben ausgebreiteten Zustand bezogen.

Der Abstand M zwischen parallel angeordneten streifenförmigen Kleberzonen bemisst sich über die Entfernung zwischen den in Querrichtung direkt benachbart verlaufenden Kanten von zwei direkt benachbarten Kleberzonen. Der Abstand N zwischen parallel angeordneten ersten Elastifizierungsmittel wird analog bestimmt, also es wird der Abstand zwischen den zwei direkt benachbarten ersten Elastifizierungsmitteln vermessen. Der Abstand P zwischen in Querrichtung beabstandet angeordneten Kleberzonenabschnitten bemisst sich über die Entfernung zwischen den in Längsrichtung direkt benachbart verlaufenden Kanten von zwei direkt benachbarten Kleberzonenabschnitten.

Der Fadendurchmesser D des ersten Elastifizierungsmittel bezieht sich hingegen auf den ungedehnten, relaxierten Zustand des ersten Elastifizierungsmittels.

Der Fadendurchmesser D kann bei Kenntnis der Materialeigenschaften des eingesetzten ersten Elastifizierungsmittels, nämlich Fadenstärke und Dichte p, nach folgender Formel berechnet werden:

$$D = 2 \times Radius\ r = 2 \times \sqrt{\frac{Fadenstärke}{\pi \bullet Dichte\,\rho}}$$

Unter der Fadenstärke wird dabei die Masse bezogen auf die Lauflänge verstanden und mit der Einheit dtex, mit 1 dtex = 1 g/10.000 m ausgedrückt. Die Dichte p als Verhältnis der Masse zum Volumen wird mit der SI- Einheit kg/m$^3$ ausgedrückt.

Im Falle eines ersten Elastifzierungsmittels, das keinen kreisförmigen Durchmesser aufweist, sondern vielmehr bandförmig erscheint, wird die größte Erstreckung zwischen den äußeren Kanten des Elastifizierungsmittels als Fadendurchmesser D herangezogen.

Bei den nachfolgend noch näher beschriebenen Quotienten G/M, G/D und S/P, wird der im Zähler genannte Parameter durch den im Nenner genannten Parameter geteilt. Hierbei handelt es um Quotienten, in welchen Breite G, Abstand M, Länge S oder Abstand P in Verhältnis zueinander gesetzt werden. Es wird dabei vorausgesetzt, dass im Zähler und Nenner stets nur Werte mit derselben Abmessungseinheit Eingang finden, so dass ein Quotient ohne Einheit resultiert.

Vorteilhafterweise kann das Deckblatt-Material und/oder das Backsheet-Material auch in Längsrichtung einen sich jeweils außerhalb von Querrändern des Absorptionskörpers erstreckenden Überhang bilden.

Unter "Überhang" wird die Erstreckung des Deckblatt-Materials und/oder des Backsheet-Materials in Querrichtung seitlich außerhalb der Längsränder des Absorptionskörpers bzw. in Längsrichtung außerhalb der Querränder des Absorptionskörpers verstanden, wobei jeweils die maximale Erstreckung, also die von den Längsrändern bzw. von den Querrändern des Absorptionskörpers am weitesten distal gelegene äußere Erstreckung des Deckblatt-Materials und/oder des Backsheet-Materials herangezogen wird. Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln sein. Es wird also so verstanden, dass auch bei Verbunden, also zusammengesetzten Deckblatt-Materialien und/oder Backsheet-Materialien, bei denen die einzelnen Lagen sich nicht kongruent überdecken, bei der Betrachtung des Überhangs die jeweils maximale, also am weitesten distal gelegene äußere Erstreckung des Verbundes bzw. der darin vorkommenden einzelnen Material-Lagen herangezogen wird.

Unter "Chassismaterialien" werden die eine Hülle des gesamten Inkontinenzartikels bzw. der einzelnen Komponenten, wie eben des Bauchabschnitts, des Rückenabschnitts und/oder des Schrittabschnitts bildenden Materialien, verstanden, wie beispielsweise das Deckblatt-Material und das Backsheet-Material des Schrittabschnitts oder die zumindest erste und zweite Lage des ersten und/oder zweiten Bauchteilabschnitts und/oder des ersten und/oder zweiten Rückenteilabschnitts.

Die ersten Elastifizierungsmittel sind erfindungsgemäß als nur ein einziges Elastifizierungsmittel innerhalb einer streifenförmigen Kleberzone abgelegt. In bevorzugter Weise sind die ersten Elastifizierungsmittel mittig innerhalb der streifenförmigen Kleberzonen abgelegt. Dadurch wird vorteilhaft eine gleichmäßige Fixierung des ersten Elastifizierungsmittels innerhalb der Klebezone und auch eine gute Fixierung der ersten und der zweiten Lage des ersten Bauchteilabschnitts und/oder des ersten Rückenteilabschnitts zueinander mittels des das erste Elastifizierungsmittel jeweils angrenzenden Bereichs der Kleberzone erreicht.

In Weiterbildung der Erfindung erweist es sich als vorteilhaft, wenn in dem ersten beinöffnungsfernen Bauchteilabschnitt und/oder in dem ersten beinöffnungsfernen Rückenteilabschnitt die Breite G der streifenförmigen Kleberzonen derart gewählt wird und die Kleberzonen in einem Abstand M derart angeordnet sind, so dass der Quotient G/M, mindestens 0,10, insbesondere mindestens 0,30, weiter insbesondere mindestens 0,50, weiter insbesondere mindestens 0,75, weiter insbesondere mindestens 1,0 , vorzugsweise höchstens 7,5, weiter vorzugsweise höchstens 6,0, weiter vorzugsweise höchstens 5,0 , weiter vorzugsweise höchstens 4,0 beträgt. Durch diese Anordnung der streifenförmigen Kleberzonen, definiert über deren Breite und deren Abstands zueinander, wird eine Stabilität des Verbundes der ersten Elastifizierungsmittel mit den Chassismaterialien erreicht, ohne den Verbund in einem zu großen Ausmaße zu versteifen oder zu weich zu belassen. Zudem wird der Einsatz an übermäßigen Einsatz an Klebermaterialien und damit auch Kosten vermieden.

In einer weiteren vorteilhaften Weiterbildung weisen die Kleberzonen eine Breite G von 2 - 15 mm, weiter vorzugsweise von 2-12 mm, weiter vorzugsweise von 2-10 mm, weiter vorzugsweise von 2-8 mm, weiter vorzugsweise von 2-6 mm auf.

In einer weiteren vorteilhaften Weiterbildung sind die Kleberzonen in einem Abstand M von 2 - 25 mm, weiter vorzugsweise von 2-20 mm, weiter vorzugsweise von 2-15 mm, weiter vorzugsweise von 2-10 mm, weiter vorzugsweise von 2-8 mm, weiter vorzugsweise 2-6 mm angeordnet.

In weiterer vorteilhafter Weise können die in Längsrichtung des Inkontinenzartikels parallel beabstandeten Kleberzonen eine unterschiedliche Breite G aufweisen. Insbesondere vorteilhaft weisen die in Richtung zur Linie L1 bzw. Linie L2 angeordneten Kleberzonen eine geringere Breite G als die in Richtung zur Hüftöffnung angeordneten Kleberzonen. Durch die in Richtung zur Hüftöffnung angeordneten breiteren Kleberzonen wird der Krafteinwirkung beim Anlegevorgang auf die insbesondere im Bereich der Hüftöffnung zwischen der ersten und zweiten Lage angeordneten ersten Elastifizierungsmittel Rechnung getragen und damit entgegengewirkt.

In einer weiteren vorteilhaften Weiterbildung werden die Breite G der Kleberzone und der Fadendurchmesser D des einzig darin angeordneten ersten Elastifizierungsmittels derart gewählt, dass der Quotient G/D mindestens 2, weiter vorzugsweise mindestens 4, weiter vorzugsweise mindestens 6, weiter vorzugsweise mindestens 8, weiter vorzugsweise mindestens 10, vorzugsweise aber höchstens 100, weiter vorzugsweise höchstens 80, weiter vorzugsweise höchstens 60, weiter vorzugsweise höchstens 50, weiter vorzugsweise höchstens 40, weiter vorzugsweise höchstens 30 beträgt. Durch die abgestimmte Auswahl der Breite G der Kleberzone und des Fadendurchmessers D des in der Kleberzone alleinig abgelegten Elastifizierungsmittels wird eine optimale Einbettung des ersten Elastifizierungsmittels innerhalb der Breite der Kleberzone und ein optimales Ausmaß eines nicht durch das erste Elastifizierungsmittel abgedeckten Bereiches der Kleberzone erhalten, welcher eine Fixierung der ersten und zweiten Lage miteinander erlaubt.

Als erste fadenförmige oder bandförmige Elastifizierungsmittel werden vorzugsweise Gummi- oder Polyetherpolyurethan- oder Polyesterpolyurethanfäden, vorzugsweise Elastikfäden wie Lycra®-, Creora (R)- oder Spandex®-Fäden eingesetzt.

Die ersten fadenförmigen oder bandförmigen Elastifizierungsmittel haben vorzugsweise eine Stärke von 300 - 1500 dtex, insbesondere von 500 - 900 dtex, weiter insbesondere von 500 - 600 dtex.

Die ersten fadenförmigen oder bandförmigen Elastifizierungsmittel haben vorzugsweise einen Fadendurchmesser D von 0,05-1,0 mm, weiter vorzugsweise von 0,05 - 0,8 mm, weiter vorzugsweise von 0,05 - 0,6 mm, weiter vorzugsweise von 0,05-0,5 mm, weiter vorzugsweise von 0,05 - 0,4 mm, weiter vorzugsweise von 0,05 - 0,3 mm.

Die ersten Elastifizierungsmittel werden im gedehnten Zustand auf die Chassismaterialien, also zwischen die erste und zweite Lage des ersten Bauchteilabschnitts und zwischen die erste und zweite Lage des ersten Rückenteilabschnitts festgelegt.

Die ersten Elastifizierungsmittel werden vorzugsweise unter einer Vorspannung von 1,5-6,0, insbesondere von 2,5 - 5,0 an den chassisbildenden Hüllmaterialien des Bauchabschnitts und Rückenabschnitts, also zwischen der ersten und zweiten Lage des ersten beinöffnungsfernen Bauchteilabschnitts und Rückenteilabschnitts fixiert. Die Vorspannung ist dabei definiert als Faktor des Dehnungsgrades gegenüber dem ungedehnten/relaxierten Zustand des Elastifizierungsmittels.

Die ersten Elastifizierungsmittel im ersten beinöffnungsfernen Bauchteilabschnitt und/oder ersten beinöffnungsfernen Rückenteilabschnitt haben vorzugsweise die gleiche

Vorspannung. Es ist indessen möglich, dass die ersten Elastifizierungsmittel in Richtung zur Hüftöffnung eine stärkere Vorspannung aufweisen oder mehrere dieser Elastifizierungsmittel mit geringerem Abstand voneinander vorgesehen sind, um eine etwas stärkere Elastifizierung im Bereich der Hüftöffnung zu realisieren.

Es hat sich insbesondere als vorteilhaft erwiesen, wenn der Abstand N der ersten Elastifizierungsmittel voneinander (also der Abstand von unmittelbar nebeneinander liegenden ersten Elastifizierungsmitteln) insbesondere 3-30 mm, weiter insbesondere 3 - 20 mm, weiter insbesondere 3 bis 15 mm, weiter insbesondere 3 bis 10 mm beträgt.

In einer bevorzugten Ausführung erstrecken sich die streifenförmigen Kleberzonen im ersten Bauchteilabschnitt bzw. im ersten Rückenteilabschnitt kontinuierlich und ohne Beabstandung in Querrichtung. Weiter vorzugsweise erstrecken sich die streifenförmigen Kleberzonen im Wesentlichen über die gesamte Querrichtung des ersten Bauchteilabschnitts bzw. des ersten Rückenteilabschnitts.

"Im Wesentlichen" bedeutet, dass die streifenförmigen Kleberzonen in einem Abstand von bis höchstens 2 % der Quererstreckung des Bauchabschnitts und/oder des Rückenabschnitts, weiter insbesondere höchstens 1 % der Quererstreckung des Bauchabschnitts und/oder des Rückenabschnitts vor den jeweiligen seitlichen Längsrandabschnitten des Bauchabschnitts und des Rückenabschnitts enden können. Weiter insbesondere enden die streifenförmigen Kleberzonen höchstens bis zu 8 mm, weiter insbesondere höchstens bis zu 5 mm vor den jeweiligen seitlichen Längsrandabschnitten des Bauchabschnitts und/oder des Rückenabschnitts.

In einer alternativen Ausführung umfassen die streifenförmigen Kleberzonen Kleberzonenabschnitten, die in Querrichtung beabstandet in einem Abstand P angeordnet sind und eine Länge S aufweisen.

Weiter vorzugsweise sind die streifenförmigen Kleberzonenabschnitte mit der Länge S derart beabstandet in Quer - oder Hüftumfangsrichtung mit Abstand P angeordnet, so dass der Quotient S/P mindestens 0,1, weiter vorzugsweise mindestens 0,3, weiter vorzugsweise mindestens 0,5, weiter vorzugsweise mindestens 1,0, weiter vorzugsweise mindestens 2,0, aber weiter vorzugsweise höchstens 15, weiter vorzugsweise höchstens 13, weiter vorzugsweise höchstens 10 beträgt. Durch diese Anordnung der streifenförmigen Kleberzonenabschnitte, definiert über deren Länge und deren Abstands zueinander, wird eine ausreichende Stabilität des Verbundes der ersten Elastifizierungsmittel mit den Chassismaterialien erreicht.

Die Anordnung der beabstandeten Kleberzonenabschnitte ist vorteilhafterweise derart, dass der Abstand P der Kleberzonenabschnitte zueinander zumindest nicht größer als die Länge S der Kleberzonenabschnitte ist.

Weiter vorzugsweise weisen die Kleberzonenabschnitte eine Länge S von mindestens 2 mm, weiter vorzugsweise von mindestens 5 mm, weiter vorzugsweise von mindestens 8 mm, weiter vorzugsweise von mindestens 10 mm auf, aber vorzugsweise von höchstens 30 mm, weiter vorzugsweise von höchstens 25 mm, weiter vorzugsweise von höchstens 20 mm auf.

Vorzugsweise sind die Kleberzonenabschnitte in einem Abstand P von mindestens 2 mm, weiter insbesondere von mindestens 4 mm, weiter insbesondere von höchstens 20 mm, weiter insbesondere von höchstens 15 mm, weiter insbesondere von höchstens 10 mm, weiter insbesondere von höchstens 8 mm angeordnet.

Der erste beinöffnungsferne Bauchteilabschnitt bzw. der erste beinöffnungsferne Rückenteilabschnitt nehmen vorzugsweise einen Anteil von 30 - 75%, weiter insbesondere von 35 - 70 %, weiter insbesondere von 35 - 65 % bezogen auf die Fläche des Bauchabschnitts bzw. des Rückenabschnitts ein.

Der Anteil der Fläche des ersten beinöffnungsfernen Bauchteilabschnitts an der Fläche des Bauchabschnitts ist vorteilhafterweise größer als der Anteil der Fläche des ersten beinöffnungsfernen Rückenteilabschnitts bezogen auf die Fläche des Rückenabschnitts. Dies ist vorteilhaft ,da beim Anlegevorgang durch den Anwender insbesondere in den vorderen Bereich eines Inkontinenzartikels, also dem Bauchabschnitt und dabei insbesondere im oberen in Richtung Hüftöffnung orientierten Bereich, also dem ersten beinöffnungsfernen Bauchteilabschnitt in die Chassismaterialien und die damit verbundenen ersten Elastifizierungsmittel gegriffen wird.

Erfindungsgemäß sind die streifenförmigen Kleberzonen in Längsrichtung des Inkontinenzartikels betrachtet in einem Abstand M zueinander angeordnet. Weiter vorzugsweise sind die streifenförmigen Kleberzonen als Kleberzonenabschnitte mit einem Abstand P in Querrichtung des Inkontinenzartikels angeordnet.

Vorzugsweise weist der erste beinöffnungsferne Bauchteilabschnitt bzw. der erste beinöffnungsferne Rückenteilabschnitt eine von Kleberzonen freie Fläche bzw. eine von Kleberzonenabschnitte freie Fläche mit einem Anteil von 20 - 80 %, insbesondere von 25-70 % , weiter insbesondere von 30 -60 % bezogen auf die Fläche des ersten beinöffnungsfernen Bauchteilabschnitts bzw. Rückenteilabschnitts auf.

Durch diesen Anteil an Klebezonen(abschnitte) freien Flächen wird vorteilhaft eine für den Anwender als angenehm gefundene Flexibilität und/oder auch Atmungsaktivität des Inkontinenzartikels bereitgestellt, wenngleich eine gemäßigte Versteifung erhalten wird.

Die Kleberzonen bzw. Kleberzonenabschnitte weisen eine adhäsive Beschichtung auf.

Die adhäsive Beschichtung ist dabei insbesondere bevorzugt mit einem Flächengewicht von 2-40 g/m$^2$, weiter insbesondere von 2-30 g/m$^2$, weiter insbesondere 2 - 20 g/m$^2$, weiter insbesondere 2-10 g/m$^2$ aufgetragen.

Die adhäsive Beschichtung umfasst einen Kleber, vorzugsweise einen Hotmeltkleber, weiter insbesondere einen hydrophoben Hotmeltkleber.

Als Kleber können beispielsweise vorzugsweise eingesetzt werden: D 9105 ZP oder LC 3001 ZP (H.B. Fuller Deutschland GmbH, An der Roten Bleiche 2-3, 21335 Lüneburg, Deutschland); H20028 oder H 2481 (Bostik Nederland B.V., Zeggeveld 10, 4705 RP Roosendaal, Niederlanden); Technomelt Q2415 oder Technomelt Q5430 (Henkel KGaA, 40191 Düsseldorf, Deutschland).

Die adhäsive Beschichtung der Kleberzone bzw. des Kleberzonenabschnitts ist vorzugsweise vollflächig innerhalb der Kleberzone bzw. des Kleberzonenabschnitts vorgesehen.

Die adhäsive Beschichtung ist auf den einander zugewandten Oberseiten der ersten und/oder zweiten Lage des ersten Bauchteilabschnitts und auf den einander zugewandten

Oberseiten der ersten und/oder zweiten Lage des ersten Rückenteilabschnitts aufgebracht. D. h., die adhäsive Beschichtung ist zumindest auf einer der genannten ersten und zweiten Lage des ersten Bauchteilabschnitts und auf einer der genannten ersten und zweiten Lage des ersten Rückenteilabschnitts aufgebracht.

In einer vorteilhaften Ausführung ist die adhäsive Beschichtung nur auf einer der genannten ersten und zweiten Lage des ersten Bauchteilabschnitts und nur auf einer der genannten ersten und zweiten Lage des ersten Rückenteilabschnitts aufgebracht. Durch das Aufbringen der adhäsiven Beschichtung auf nur jeweils einer der ersten und zweiten Lage, zwischen denen das erste Elastifizierungsmittel angeordnet ist, kann der Prozess des Auftrags des Klebemittels technisch einfacher gelöst werden.

In einer alternativen Ausführung kann die adhäsive Beschichtung auf der ersten und auf der zweiten Lage des ersten Bauchteilabschnitts und/oder des ersten Rückenteilabschnitts aufgebracht sein. Hierbei sind die jeweils erste und zweite Lage mit einem im Wesentlichen deckungsgleichen Auftragsmuster an adhäsiver Beschichtung versehen, welche dann in der Laminatanordnung mit dem mittig gelegenen ersten Elastifizierungsmittel abgestimmt aufeinander zu liegen kommen. Durch die Bereitstellung von adhäsiver Beschichtung auf beiden Lagen wird das erste Elastifizierungsmittel förmlich in einen Mantel an adhäsiver Beschichtung eingebettet, wodurch eine sehr gute Fixierung des ersten Elastifizierungsmittels an die erste und zweite Lage erhalten wird. Vorteilhafterweise werden dabei die bereits voranstehend erläuterten negativen Auswirkungen einer Einzelstrangbeleimung vermieden.

Bei dieser alternativen Ausführung kann vorteilhafterweise die pro Lage eingesetzte Menge an Klebemittel, also das Flächengewicht, im Vergleich zu einer adhäsiven Beschichtung auf nur einer Lage reduziert werden, wie insbesondere halbiert werden. Es ist aber durchaus denkbar, dass das Flächengewicht der adhäsiven Beschichtung für die erste und die zweite Lage sich unterscheiden. Dies kann abhängig von den eingesetzten Chassismaterialien der ersten und zweiten Lage sein.

Die adhäsive Beschichtung der Kleberzone bzw. des Kleberzonenabschnitts kann mit verschiedenen im Stand der Technik bekannten Verfahren, wie Kontaktauftragsverfahren, kontaktloses Auftragsverfahren, wie beispielsweise auch durch Sprühen, auf die jeweilige erste und/oder zweite Lage des ersten Bauchteilabschnitts bzw. des ersten Rückenteilabschnitts aufgebracht werden.

Bevorzugt wird die adhäsive Beschichtung zur Bereitstellung der Kleberzonen bzw. Kleberzonenabschnitte auf die erste und/oder zweite Lage des ersten Bauchteilabschnitts bzw. des ersten Rückenteilabschnitts im Kontaktverfahren aufgebracht. Im Kontaktverfahren werden die entsprechende jeweilige erste und/oder zweite Lage des ersten Bauchteilabschnitts bzw. des ersten Rückenteilabschnitts direkt streifenförmig mit der adhäsiven Beschichtung versehen, indem der Kleberauftragskopf und das Chassismaterial in einer Relativbewegung zueinander geführt werden. Mit dem Kontaktverfahren kann die gewünschte Streifenform mit den gewünschten Dimensionen der Kleberzone (Breite, Abstand) vorteilhafter gegenüber kontaktlosen Verfahren, wie Sprühen bereitgestellt, da bei letzterem Verfahren die Gefahr der Vernebelung des Klebemittels und damit ein ungenauerer Auftrag des Klebemittels besteht.

In Weiterbildung der Erfindung erweist es sich als vorteilhaft, wenn der vordere Überlappungsbereich von Schrittabschnitt zum Bauchabschnitt wenigstens 12 % , insbesondere 15 - 40 %, weiter insbesondere 15 - 35 % und weiter insbesondere 15 - 25 % der Fläche des Bauchabschnitts einnimmt und der hintere Überlappungsbereich von Schrittabschnitt zum Rückenabschnitt wenigstens 20 % , insbesondere 20 - 40 %, weiter insbesondere 20 - 35 % und weiter insbesondere 22 - 32 % der Fläche des Rückenabschnitts einnimmt..

In vorteilhafter Weise überlappt der Schrittabschnitt den Bauchabschnitt mit einer Fläche von 25.000 - 45.000 mm$^2$.

In vorteilhafter Weise überlappt der Schrittabschnitt den Rückenabschnitt mit einer Fläche von 35.000 - 65.0000 mm$^2$, insbesondere von 40.000 - 55.000 mm$^2$.

Die Überlappung des Schrittabschnitts mit dem Rückenabschnitt ist in vorteilhafter Weise größer als die Überlappung des Schrittabschnitts mit dem Bauchabschnitt.

Erfindungsgemäß sind der Schrittabschnitt und der Bauchabschnitt bzw. der Rückenabschnitt unlösbar miteinander verbunden. Es erweist sich des Weiteren als besonders vorteilhaft, dass der Schrittabschnitt mittels eines nicht vollflächigen Kleberauftrags mit dem Bauchabschnitt und/oder mit dem Rückenabschnitt verbunden werden kann. Es hat sich nämlich gezeigt, dass bei Verwendung eines nicht vollflächigen Kleberauftrags die Eigenschaften der Chassismaterialien in geringerem Maße beeinflusst werden als wenn ein vollflächiger Kleberauftrag zur Ausbildung der Verbindung von Schrittabschnitt und Bauchabschnitt bzw. Schrittabschnitt und Rückenabschnitt verwendet wird. Bei einem nicht vollflächigen Kleberauftrag kann es sich beispielsweise um ein streifenförmiges Muster, um eine stegförmige kontinuierliche oder nicht kontinuierliche Gitterstruktur oder um inselförmige Bereiche oder aber um eine streifenförmige oder spiralförmig angeordnete Kleberstruktur handeln.

Insbesondere bevorzugt sind bei dem Inkontinenzartikel in Höschenform auch in dem zweiten beinöffnungsnahen Bauchteilabschnitt und/oder in dem zweiten beinöffnungsnahen Rückenteilabschnitt zweite insbesondere fadenförmige oder bandförmige Elastifizierungsmittel vorgesehen, insbesondere derart, dass die zweiten Elastifizierungsmittel den jeweiligen zweiten Bauchteilabschnitt oder Rückenteilabschnitt durchgehend elastifizieren.

Die zweiten Elastifizierungsmittel erstrecken sich bevorzugt ausgehend von den beiden Seitennahtbereichen des zweiten Bauchteilabschnitts und/oder des zweiten Rückenteilabschnitts in Richtung auf eine Längsmittelachse des Inkontinenzartikels und insbesondere bevorzugt verlaufen die zweiten Elastifizierungsmittel dabei bogenförmig sich auffächernd mit zunehmendem Abstand voneinander.

Vorteilhafterweise sind die zweiten Elastifizierungsmittel so ausgebildet, dass die bei flächenhafter Dehnung dieses zweiten Bauchteilabschnitts und/oder des zweiten Rückenteilabschnitts auftretende Rückstellkraft geringer ist als bei flächenhafter Dehnung in dem entsprechenden ersten Bauchteilabschnitt bzw. ersten Rückenteilabschnitt, in welchem nur die ersten Elastifizierungsmittel vorgesehen sind. Hierdurch wird ein besserer Tragekomfort des Inkontinenzartikels erreicht, weil der Inkontinenzartikel in dem jeweiligen beinöffnungsnahen Bereich der Körperform entsprechend auch in größerem Umfang gedehnt werden kann ohne dass es gleich zu einer unangenehmen Erhöhung der Rückstellkräfte, welche dann insbesondere bei hoher Mobilität des Benutzers durch die Relativbewegungen von Inkontinenzartikel zu Anwender zu unangenehmen bis hin zu medizinisch problematischen Hautreizungen führen können.

Bei der Rückstellkraft handelt es sich um diejenige Kraft, welche der erste bzw. zweite Bauchabteilschnitt bzw. Rückenabteilschnitt einer flächenhaften Dehnung in Richtung des Verlaufs der ersten und/oder zweiten Elastifizierungsmittel entgegensetzt.

Zur Bestimmung der Rückstellkräfte können die zu vermessenden Bereiche des Chassismaterial direkt, gleichsam zerstörungsfrei zwischen zwei Klemmbacken von definierter, gleicher Klemmbackenbreite fest eingespannt und die Rückstellkräfte bei einer definierten, den Gebrauchszustand simulierenden Dehnung der zu messenden Bereiche um insbesondere 30% oder 50% oder 80% der Ausgangslänge (des Klemmbackenabstandes bei Fixieren des zu messendes Bereiches in ungespanntem Zustand) ermittelt werden. Die Klemmbacken sollten möglichst viele, zumindest jedoch zwei nebeneinander angeordnete Elastifizierungsmittel des zu messenden Bereiches fixieren und im Wesentlichen senkrecht zum Verlauf der Elastifizierungsmittel orientiert sein, so dass die Dehnung zwischen den Klemmen im Wesentlichen in Richtung des Verlaufs der Elastifizierungsmittel erfolgt.

Hierfür ist ein Bereich im zweiten beinöffnungnahen Bauchteilabschnitt bzw. im zweiten beinöffnungsnahen Rückenteilabschnitt, in welchem die zweiten Elastifizierungsmittel sich in Richtung auf die Längsmittelachse auffächern, vorzugsweise so ausgebildet, dass bei flächenhafter Dehnung dieses Bereichs die dabei auftretende Rückstellkraft in Richtung auf den Schrittabschnitt abnimmt. Betrachtet man also diesen zweiten beinöffnungsnahen Bauchteilabschnitt und Rückenteilabschnitt, und zwar in einer Richtung ausgehend von dem jeweiligen Seitennahtbereich in Richtung auf den Schrittabschnitt, also in Richtung auf eine Längsmittelachse des Inkontinenzartikels und gewissermaßen in Richtung der bogenförmigen Auffächerung der zweiten Elastifizierungsmittel, so wird die bei flächenhafter Dehnung auftretende Rückstellkraft in dieser Richtung reduziert. Es handelt sich also hierbei um diejenige Kraft, welche der zweite Bauchabteilschnitt und der zweite Rückenteilabschnitt einer flächenhaften Dehnung entgegensetzen. Eine Abnahme dieser Rückstellkraft, die sich dann naturgemäß auf den Benutzer überträgt, ist mit einer erheblichen Verbesserung des Tragekomforts des Inkontinenzartikels verbunden.

Es hat sich insbesondere als vorteilhaft erwiesen, wenn ein minimaler Abstand der zweiten Elastifizierungsmittel voneinander (Abstand von unmittelbar nebeneinander liegenden Elastifizierungsmitteln) in den Seitennahtbereichen 3 bis 8 mm, insbesondere 3 bis 7 mm und weiter insbesondere 3 bis 6 mm beträgt.

Des Weiteren hat es sich als vorteilhaft erwiesen, wenn ein maximaler Abstand der zweiten Elastifizierungsmittel voneinander (Abstand von unmittelbar nebeneinander liegenden Elastifizierungsmitteln) an einem Absorptionskörperrand oder an einem Längsrand des Schrittabschnitts 7 bis 35 mm, insbesondere 10 bis 32 mm und weiter insbesondere 12 bis 30 mm beträgt.

Des Weiteren hat es sich als vorteilhaft erwiesen, wenn die zweiten Elastifizierungsmittel einen Auffächerungsgrad F

$$F=(A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am Längsrand des Schrittabschnitts oder am Absorptionskörperrand, und B als der minimale Abstand insbesondere im Seitennahtbereich. Es wurde auch erkannt, dass es sich als vorteilhaft erweist, wenn der Auffächerungsgrad F der zweiten Elastifizierungsmittel im Rückenabschnitt größer ist als im Bauchabschnitt.

Aufgrund der naturbedingten Körperformen im Rückenbereich bzw. Bauchbereich eines Benutzers erweist es sich als vorteilhaft, wenn der maximale Abstand der zweiten Elastifizierungsmittel voneinander an einem Absorptionskörperrand im Rückenabschnitt größer ist als im Bauchabschnitt.

Es wäre durchaus denkbar, dass die zweiten Elastifizierungsmittel von dem einen Seitennahtbereich zu dem anderen Seitennahtbereich durchgehend verlaufen, was insbesondere die Einbringung in einem kontinuierlichen Herstellungsverfahren im Vergleich zu einem "cut- and- place"- Prozess vereinfacht. Infolge der Überdeckung des Schrittabschnitts mit dem Bauchabschnitt und mit dem Rückenabschnitt kann es je nach Gestaltung auch zu einer Überlappung oder Überdeckung des materialreichen Absorptionskörpers mit dem Bauchabschnitt und/ oder dem Rückenabschnitt kommen und somit auch mit demjenigen zweiten beinöffnungsnahen Bauchteilabschnitts und Rückenteilabschnitt, in dem die zweiten Elastifizierungsmittel verlaufen. Der materialreiche Absorptionskörper behindert dabei üblicherweise eine elastische Dehnbarkeit der Chassismaterialien. Außerdem ist es nicht unbedingt vorteilhaft, wenn der materialreiche Absorptionskörper mit zusätzlichen Spannungskräften beaufschlagt wird. Deshalb kann es sich als vorteilhaft erweisen, wenn die zweiten Elastifizierungsmittel in einem Überlappungsbereich mit dem Absorptionskörper des Schrittabschnitts hinsichtlich ihrer elastischen Eigenschaften deaktiviert sind. Diese Deaktivierung kann beispielsweise durch eine Anzahl von Trennschnitten durch die zweiten Elastifizierungsmittel im Bereich der Überdeckung mit dem Absorptionskörper realisiert werden, wobei auch andere Trennverfahren, wie z. Bsp. mittels Ultraschallschweißen oder Laser denkbar sind.

Es wurde bereits darauf hingewiesen, dass die zweiten Elastifizierungsmittel ungeachtet der vorzugsweise herbeizuführenden Spannungsverhältnisse entsprechend dem Weg ihres aufgefächerten Verlaufs bei der Herstellung des Inkontinenzartikels einer stärkeren Dehnung und damit einer höheren Vorspannung ausgesetzt sein können als in einem nicht aufgefächerten Bereich, in welchem sie sich im Wesentlichen äquidistant zueinander und in der Maschinenrichtung erstrecken. Diese stärkere Vorspannung kann sich typischerweise infolge des Einbringens der zweiten Elastifizierungsmittel in einem üblichen und daher nicht im Einzelnen zu beschreibenden Stretch-Bond-Verfahren ergeben.

Die Festlegung der zweiten Elastifizierungsmittel im zweiten beinöffnungsnahen Bauchteilabschnitt und/oder im zweiten beinöffnungsnahen Rückenteilabschnitt erfolgt insbesondere derart, dass im zweiten beinöffnungsnahen Bauchteilabschnitt und/oder im zweiten beinöffnungsnahen Rückenteilabschnitt jeweils mindestens eine erste Lage und eine zweite Lage vorgesehen sind, zwischen denen die zweiten Elastifizierungsmittel vorzugsweise mittels eines vollflächig auf die erste und/oder zweite Lage aufgebrachten Klebemittels festgelegt werden.

Hinsichtlich der Gesamtabmessungen des Inkontinenzartikels erweist es sich als vorteilhaft, wenn der Abstand (C) des schrittzugewandten innersten zweiten Elastifizierungsmittels des Bauchabschnitts von dem entsprechenden schrittzugewandten innersten zweiten Elastifizierungsmittel des Rückenabschnitts 250 bis 420 mm beträgt.

Der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts beträgt vorzugsweise 2-40 mm, weiter vorzugsweise 3-30 mm, insbesondere vorzugsweise 4 - 15mm.

Als zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora (R)- oder Spandex®-Fäden eingesetzt. Die zweiten Elastifizierungsmittel haben vorzugsweise eine Stärke von 300-1500 dtex, insbesondere von 500 - 900 dtex, weiter insbesondere von 500 - 600 dtex.

Die zweiten Elastifizierungsmittel werden vorzugsweise unter einer Vorspannung von 1,5 - 6,0, insbesondere von 2,5 - 5,0 an den chassisbildenden Hüllmaterialien des zweiten Bauchteilabschnitts und zweiten Rückenteilabschnitts fixiert.

Die Vorspannung ist dabei definiert als Faktor des Dehnungsgrades gegenüber dem ungedehnten/relaxierten Zustand des Elastifizierungsmittels.

Die Erstreckung des Bauchabschnitts im Seitennahtbereich mit einer Länge B in Längsrichtung und die Erstreckung des Rückenabschnitts im Seitennahtbereich mit einer Länge R in Längsrichtung beträgt vorteilhafterweise wenigstens 100 mm, insbesondere wenigstens 150 mm und insbesondere 150 mm bis 220 mm.

Die Erstreckung des ersten beinöffnungsfernen Bauchteilabschnitts im Seitennahtbereich mit einer Länge B1 in Längsrichtung beträgt vorteilhafterweise wenigstens 60 mm, insbesondere wenigstens 80 mm und insbesondere 80 mm bis 160 mm. Die Erstreckung des ersten beinöffnungsfernen Rückenteilabschnitts im Seitennahtbereich mit einer Länge R1 in Längsrichtung beträgt vorteilhafterweise wenigstens 60 mm, insbesondere wenigstens 80 mm, weiter insbesondere 80 mm bis 160 mm.

Der minimale Abstand des Bauchabschnitts und des Rückenabschnitts in Längsrichtung voneinander beträgt vorteilhafterweise 250 bis 400 mm.

Die maximale Erstreckung des Schrittabschnitts in Querrichtung, also die größte Breite E, beträgt vorteilhafterweise wenigstens 200 mm, insbesondere 200 bis 350 mm, weiter insbesondere 250 bis 320 mm. Die größte Breite des Schrittabschnitt weist der Schrittabschnitt vorteilhafterweise innerhalb der Überlappungsbereiche von Schrittabschnitt mit Bauchabschnitt und/oder Rückenabschnitt auf.

Dadurch, dass der Schrittabschnitt mit einer sehr ausladenden Breite in Querrichtung von wenigstens 200 mm ausgebildet wird, eben insbesondere im Überlappungsbereich, kann vorteilhaft eine stabile Verbindung zwischen dem Schrittabschnitt mit Bauchabschnitt bzw. Rückenabschnitt erreicht werden.

Des weiteren erweist es sich als vorteilhaft, wenn der Überhang des Backsheet-Materials und/oder des Deckblatt-Materials in Querrichtung in der Summe, also beidseits der Längsränder des Absorptionskörpers wenigstens 25%, insbesondere 25 - 50 %, weiter insbesondere 30 - 45 % und weiter insbesondere 35 - 45 % bezogen auf die größte Breite E des Schrittabschnitts beträgt.

Der verhältnismäßig große Überhang von Backsheet-Material und/oder Deckblatt-Material beidseits der Absorptionskörpers bedeutet also einen breiten Schrittabschnitt mit einem verhältnismäßig schmalen Absorptionskörper.

Hierdurch wird ein großer Überlappungsbereich zwischen Schrittabschnitt und Bauchabschnitt einerseits und zwischen Schrittabschnitt und Rückenabschnitt andererseits realisiert. Hierdurch ist es möglich, entlang der Beinöffnungen erstreckte Beinelastifizierungsmittel in dem Schrittabschnitt vorzusehen, die einen verhältnismäßig großen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragekomfort des Inkontinenzartikels auswirkt.

Vorteilhafterweise bilden das Deckblatt-Material und/oder das Backsheet-Material zusätzlich in Längsrichtung einen sich jeweils außerhalb von Querrändern des Absorptionskörpers erstreckenden Überhang.

Vorteilhafterweise haben das Backsheet-Material und das Deckblatt-Material dieselbe Erstreckung in Querrichtung bzw. optional in Längsrichtung. Sie sind kongruent, also deckungsgleich zueinander.

Weiter vorteilhaft ist aber auch, wenn das Backsheet-Material und Deckblatt-Material nicht kongruent zueinander sind.

Insbesondere vorteilhafterweise hat das Backsheet-Material eine im Vergleich zum Deckblatt-Material schmälere Erstreckung in Querrichtung bzw. in Längsrichtung. Damit wird das den Anwender im Tragekomfort möglicherweise störende Backsheet-Material, wie beispielsweise eine Folie, durch das Deckblatt-Material, das insbesondere ein Vliesmaterial ist, überdeckt.

Die chassisbildenden Materialien von Bauchabschnitt und/oder Rückenabschnitt und damit die ersten und zweiten Lagen des ersten und zweiten Bauchteilabschnitts und/oder die ersten und zweiten Lagen des ersten und zweiten Rückenteilabschnitts umfassen vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese. Besonders bevorzugt umfasst das chassisbildende Material von Bauchabschnitt und/oder Rückenabschnitt Spinnvlies. Die für Bauchabschnitt und/oder Rückenabschnitt und damit für die entsprechenden Bauchteilabschnitte bzw. Rückenteilabschnitte eingesetzten Vliesstoffmaterialien haben vorteilhafterweise ein Flächengewicht von 10-30 g/m$^2$, weiter vorzugsweise von 15-25 g/m$^2$. Besonders bevorzugt umfassen der Bauchabschnitt und der Rückenabschnitt ein Spinnvlies aus Polypropylen, insbesondere mit einem Flächengewicht von 15-25 g/m$^2$.

Die chassisbildenden Hüllmaterialien des Schrittabschnitts sind weiter vorteilhaft ausgebildet: Das Backsheet umfasst insbesondere eine Folie, insbesondere eines Flächengewichts von 10-40 g/m$^2$. Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300 g/m$^2$/24h, weiter insbesondere wenigstens 1000 g/m$^2$/24h, weiter insbesondere wenigstens 2000 g/m$^2$/24h, weiter insbesondere wenigstens 3000 g/m$^2$/24h, weiter insbesondere wenigstens 4000 g/m$^2$/24h, weiter insbesondere höchstens 6000 g/m$^2$/24h gemessen nach DIN 53122-1 (Ausgabe: 2001-08).
Die Folie kann vorteilhaft auch mit einer Vliesbeschichtung versehen sein, was eine textile Anmutung der körperabgewandten Außenseite des Inkontinenzartikels vermitteln kann. Die Vliesbeschichtung besteht vorzugsweise aus einem Vliesstoff, insbesondere einem Spinnvlies aus Polypropylen, insbesondere mit einem Flächengewicht von 7-25 g/m$^2$, 10-20 g/m$^2$, insbesondere von 12-17 g/m$^2$.

Das Deckblatt- Material umfasst vorzugsweise Vliesstoffmaterialien, wie Spinnvliese (S), Meltblownvliese (M), SM- Vliese, SMS- Vliese, SMMS- Vliese, Kardenvliese oder Through Air bonded Kardenvliese.
Das Deckblatt- Material kann dabei vorzugsweise nur aus Topsheet- Material gebildet sein. Weiter vorzugsweise kann das Deckblatt- Material ein Verbund aus Topsheet- Material und Barrieremittel sein.
Entsprechend der Funktionalität werden nachstehend genannte vorteilhafte Materialien eingesetzt. Das Topsheet- Material umfasst vorzugsweise Vliesstoffmaterialien, wie Spinnvliese, Kardenvliese oder Through Air bonded Kardenvliese. Besonders bevorzugt umfasst das Topsheet- Material Spinnvlies. Die für das Topsheet eingesetzten Vliesstoffmaterialien haben weiter vorteilhafterweise ein Flächengewicht von 5- 20 g/m$^2$, 8- 20 g/m$^2$, weiter vorzugsweise von 10- 18 g/m$^2$, insbesondere vorzugsweise von 12- 16 g/m$^2$. Besonders bevorzugt umfasst das Topsheet ein hydrophilisiertes Spinnvlies, insbesondere aus Polypropylen, insbesondere mit einem Flächengewicht von 12- 16 g/m$^2$. Das Barrieremittel- Material umfasst vorzugsweise Vliesstoffmaterialien, wie Spinnvliese, Meltblownvliese, Kardenvliese oder Through Air bonded Kardenvliese. Besonders bevorzugt umfasst das Barrieremittel- Material einlagige oder mehrlagige Vliese. Insbesondere bevorzugt umfasst das Barrieremittel- Material Laminate aus einer oder mehreren Spinnvlies (S) und/ oder Meltblown (M)- Vlieslagen, insbesondere Laminate aus Spinnvlies und Meltblown- Vlieslagen, mit insbesondere einer Spinnvlies- Lage als äußere Lage des Laminats, mit insbesondere mehreren Meltblown- Vlieslagen als innere Lagen, wie beispielsweise insbesondere SMS- Laminate, SMMS- Laminate, SMMMS- Laminate. Die Meltblown- oder Spinn- Vlieslagen sind insbesondere auf Basis von Polyolefinen, wie beispielsweise Polyethylen oder Polypropylen Derartige Materialien sind kostengünstig und aufgrund ihrer inhärent hydrophoben Eigenschaft geeignet, flüssigkeitsretardierend zu wirken. Die für das Barrieremittel eingesetzten Vliesstoffmaterialien haben weiter vorteilhafterweise ein Flächengewicht von 5- 20 g/m$^2$, vorzugsweise von 8- 20 g/m$^2$, weiter vorzugsweise von 10- 18 g/m$^2$. Besonders bevorzugt umfasst das Barrieremittel ein Laminat aus Spinnvlies und Meltblown- Vlieslagen, insbesondere aus Polypropylen, insbesondere mit einem Flächengewicht von 10- 18 g/m$^2$.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskern außerdem superabsorbierende Materialien (SAP), insbesondere auf Basis oberflächenvernetzter, teilneutralisierter Polyacrylate.

Der Schrittabschnitt bzw. die Längsränder des Schrittabschnitts, welche die Beinöffnungen begrenzen, sind vorteilhafterweise bogenförmig konturiert ausgebildet.

Das erfindungsgemäße Verfahren:

Ebenso erfindungsgemäß ist das Verfahren gemäß den Merkmalen nach Anspruch 14 zum Herstellen von Inkontinenzartikel in Höschenform für die Aufnahme von Körperausscheidungen:
Der Inkontinenzartikel mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind. Zudem hat der Inkontinenzartikel einen einen Absorptionskörper aufweisenden Schrittabschnitt, wobei der Schrittabschnitt mit einem bauchseitigen Längsende und einem rückenseitigen Längsende sich in einer Längsrichtung zwischen dem Bauchabschnitt mit einem schrittzugewandten Querrand und dem Rückenabschnitt mit einem schrittzugewandten Querrand erstreckt.

Der Schrittabschnitt überlappt in einem vorderen Überlappungsbereich den Bauchabschnitt und ist an den Bauchabschnitt unlösbar angefügt und der Schrittabschnitt überlappt in einem hinteren Überlappungsbereich den Rückenabschnitt überlappt und ist an den Rückenabschnitt unlösbar angefügt.

Der Schrittabschnitt umfasst ein flüssigkeitsundurchlässiges Backsheet-Material und ein Deckblatt-Material, zwischen denen der Absorptionskörper angeordnet ist, wobei das Deckblatt-Material und/oder das Backsheet-Material in Querrichtung einen sich jeweils außerhalb von Längsrändern des Absorptionskörpers erstreckenden Überhang bilden.

Der Schrittabschnitt als auch der Bauchabschnitt und der Rückenabschnitt begrenzen die Beinöffnungen des Inkontinenzartikels.

Das bauchseitige Längsende des Schrittabschnitts erstreckt sich in Längsrichtung bis zu einer parallel zur Querrichtung verlaufenden gedachten Line L1, und das das rückenseitige Längsende des Schrittabschnitts erstreckt sich in Längsrichtung bis zu einer parallel zur Querrichtung verlaufenden gedachten Line L2, wobei die Linie L1 den Bauchabschnitt in einen ersten beinöffnungsfernen Bauchteilabschnitt und einen zweiten beinöffnungsnahen Bauchteilabschnitt und die Linie L2 den Rückenabschnitt in einen ersten beinöffnungsfernen Rückenteilabschnitt und einen zweiten beinöffnungsnahen Rückenteilabschnitt teilt.

Im ersten Bauchteilabschnitt und dem ersten Rückenteilabschnitt sind erste fadenförmige oder bandförmige Elastifizierungsmittel mit einem Fadendurchmesser D vorgesehen, die sich in einem Abstand N voneinander und parallel zueinander in Quer- oder Hüftumfangsrichtung erstrecken und so den ersten Bauchteilabschnitt und den ersten Rückenteilabschnitt flächenhaft elastifizieren, wobei der erste Bauchteilabschnitt und der erste Rückenteilabschnitt jeweils mindestens eine erste Lage und eine zweite Lage aufweisen, zwischen denen die ersten Elastifizierungsmittel festgelegt sind.

Das erfindungsgemäße Verfahren für das Festlegen der ersten Elastifizierungsmittel weist die folgenden Verfahrensschritte auf:

- Zuführen zweier die jeweils erste Lage des ersten Bauchteilabschnitts und des ersten Rückenteilabschnitts des Inkontinenzartikels bildenden Teilbahnen,
- Zuführen zweier die jeweils zweite Lage des ersten Bauchteilabschnitts und des ersten Rückenabteilschnitts bildenden Teilbahnen,
- Aufbringen einer adhäsiven Beschichtung in Form von streifenförmigen Kleberzonen in Maschinenrichtung mit einer Breite G parallel und in einem Abstand M zueinander auf mindestens eine der beiden den ersten Bauchteilabschnitt bildenden Teilbahnen und auf mindestens eine der beiden den ersten Rückenteilabschnitt bildenden Teilbahnen,
- Zuführen von ersten Elastifizierungsmittel mit einem Fadendurchmesser D in Maschinenrichtung parallel und einem Abstand N zueinander, wobei der Abstand N der zugeführten benachbarten ersten Elastifizierungsmittel größer ist als der Abstand M benachbarter Kleberzonen, und Ablegen von jeweils einem einzigen ersten Elastifizierungsmittel in Maschinenrichtung innerhalb einer einzelnen Kleberzone auf den den ersten Bauchteilabschnitt und auf den den ersten Rückenteilabschnitt bildenden Teilbahnen,
- Verbinden der ersten Elastifizierungsmittel mit den Teilbahnen.

Zum Erhalt eines einzelnen Inkontinenzartikels mit dreikomponentigen Aufbau werden vorteilhaft die weiteren Verfahrensschritte durchgeführt:

- Zuführen von Schrittabschnitten und Zusammenführen der Schrittabschnitte mit den den ersten Bauchteilabschnitt bildenden Teilbahnen und mit den den ersten Rückenabschnitt bildenden Teilbahnen, derart, dass die Schrittabschnitte in einer Längsrichtung quer zur Maschinenrichtung einenends mit der einen Teilbahn und anderenends mit der anderen Teilbahn im vorderen und hinteren Überlappungsbereich plan angeordnet sind und die Schrittabschnitte mit einem Abstand in der Maschinenrichtung voneinander angeordnet sind, und Fixieren der Schrittabschnitte und Teilbahnen in den Überlappungsbereichen,
- Falten um eine in Maschinenrichtung verlaufende Faltlinie, derart, dass die eine Teilbahn über die andere Teilbahn zu liegen kommt,
- Fügen der übereinander liegenden Teilbahnen quer zur Maschinenrichtung in Abständen voneinander zur Ausbildung von Seitennahtbereichen der herzustellenden Inkontinenzartikel, und Erhalt von einen Bauchabschnitt, einen Rückenabschnitt und einen dazwischen angeordneten Schrittabschnitt aufweisenden Produkten,
- Ausführen eines Trennschnitts quer zur Maschinenrichtung und Erhalt von vereinzelten fertigen Inkontinenzartikein.

Vorteilhafterweise werden nur jeweils auf die die erste oder die zweite Lage bildenden Teilbahnen des ersten Bauchteilabschnitts und des ersten Rückenteilabschnitts eine adhäsive Beschichtung aufgebracht. Bei dieser Ausführung gestaltet sich die Prozessführung technisch einfach.

Es ist aber auch die Alternative denkbar, dass auf beiden Teilbahnen, welche die erste und die zweite Lage des ersten Bauchteilabschnitts und die erste und zweite Lage des ersten Rückenteilabschnitts bilden, Kleberzonen aufgebracht werden. Dazu wird auf den zugewandten Oberseiten der beiden die jeweils erste und zweite Lage bildenden Teilbahnen eine adhäsive Beschichtung in Form von parallel zueinander angeordneten Kleberzonen aufgebracht. Dabei werden die die erste und zweite Lage bildenden Teilbahnen mit deckungsgleichen Auftragsmustern der adhäsiven Beschichtung versehen. Diese Teilbahnen werden dann bei der Laminatbildung mit den ersten Elastifizierungsmittel mit den deckungsgleichen Auftragsmustern entsprechend dem Auftragsmuster abgestimmt zueinander in Kontakt gebracht. In der Ausführungsform der adhäsiven Beschichtung auf beiden Teilbahnen kann pro Teilbahn und pro Kleberzone eine geringere Klebstoffmenge, d.h. ein geringeres Flächengewicht der adhäsiven Beschichtung, eingesetzt werden.

In Weiterbildung der Erfindung werden zusätzlich zu den ersten Elastifizierungsmittel zweite Elastifizierungsmittel zugeführt.

Vorzugsweise werden die zweiten Elastifizierungsmittel auf den vorgegebenen den ersten Bauchteilabschnitt und/oder den ersten Rückenteilabschnitt bildenden Teilbahnen in einem von ersten Elastifizierungsmittel freien Bereich aufgebracht.

Alternativ zu den bereits vorgegebenen den den ersten Bauchteilabschnitt und/oder den ersten Rückenteilabschnitt bildenden Teilbahnen, und/oder auch in Ergänzung dazu, können die zweiten Elastifizierungsmittel auf jeweils einer weiteren zugeführten den Bauchabschnitt und/oder den Rückenabschnitt bildenden Teilbahn aufgebracht werden.

Vorzugsweise werden die zweiten Elastifizierungsmittel derart zugeführt, dass sie in dem späteren beinöffnungsnahen zweiten Bauchteilabschnitt und/oder zweiten Rückenteilabschnitt zu liegen kommen.

Die Aufteilung des Bauchabschnitts und Rückenabschnitts in einen beinöffnungsfernen ersten Bauchteilabschnitt und einen beinöffnungsnahen zweiten Bauchteilabschnitt bzw. in einen beinöffnungsfernen ersten Rückenteilabschnitt und einen beinöffnungsnahen zweiten Rückenteilabschnitt wird durch die Anordnung des Schrittabschnitts mit seinem bauchseitigen Längsende auf dem Bauchabschnitt und mit seinem rückenseitigen Längsende auf dem Rückenabschnitt erhalten, indem eine am jeweiligen Längsende in Querrichtung parallel verlaufende gedachte Linie L1 bzw. L2 den Bauchabschnitt bzw. den Rückenabschnitt teilt.

Vorzugsweise werden die zweiten Elastifizierungsmittel auf beiden weiteren den Bauch- und Rückenabschnitt bildenden Teilbahnen aufgebracht.

Vorzugsweise werden die zweiten Elastifizierungsmittel derart zugeführt, so dass sie sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.

Zur Festlegung der zweiten Elastifizierungsmittel im zweiten beinöffnungsnahen Bauchteilabschnitt und/oder im zweiten beinöffnungsnahen Rückenteilabschnitt werden die den zweiten Bauchteilabschnitt und/oder zweiten Rückenteilabschnitt bildende Teilbahnen auf den den zweiten Elastifizierungsmitteln zugewandten Oberseiten vorzugsweise mit einem vollflächig aufgebrachtem Klebemittel versehen.

Die Teilbahnen der ersten und zweiten Lage des späteren ersten Bauchteilabschnitts und/oder ersten Rückenteilabschnitts und/oder des zweiten Bauchabteilschnitts und/oder des zweiten Rückenabteilschnitts sind vorzugsweise aus den Chassismaterialien und mit den Flächengewichten, so wie voranstehend beschrieben.

Die ersten Elastifizierungsmittel sind vorzugsweise aus den Materialien und weisen vorzugsweise den Fadendurchmesser D auf, so wie voranstehend beschrieben.

Die adhäsive Beschichtung wird vorzugsweise in Form der Klebemittel und mit den Flächengewichten aufgetragen, so wie voranstehend beschrieben.

Die Kleberzonen bzw. Kleberzonenabschnitte werden durch das Auftragen der adhäsiven Beschichtung erhalten, vorzugsweise mit einer Breite B, in einem Abstand M, mit einer Länge S und/oder in einem Abstand P, wie voranstehend beschrieben. Die ersten Elastifizierungsmittel sind vorzugsweise in einem Abstand N, wie voranstehend beschrieben, angeordnet. Vorzugsweise ist die Anordnung der Kleberzonen bzw. Kleberzonenabschnitte bzw. der ersten Elastifizierungsmittel derart, dass die Quotienten G/M, G/D und/oder S/P, wie voranstehend beschrieben, erhalten werden.

Zum Auftragen der adhäsiven Beschichtung werden die die erste und/oder zweite Lage bildenden Teilbahnen, insbesondere die Teilbahnen des späteren ersten Bauchteilabschnitt und ersten Rückenteilabschnitts, kontinuierlich mit einer Geschwindigkeit v1 einer Beleimungsstation zugeführt. Vorzugweise wird die entsprechende Teilbahn mittels Rollen an die Beleimungsstation vorbeigeführt. Vorteilhafterweise wird die Teilbahn derart an die Beleimungsstation mit einem Austrittsbereich für das Klebemittel vorbeigeführt, dass die Teilbahn den Austrittsbereich berührt und das Klebemittel somit in einem Kontaktverfahren aufgebracht wird.

Vorteilhafterweise ist der Austrittsbereich des Auftragskopfs mit offenen und verschlossenen Bereichen ausgestattet bzw. kann mit einer Einlegemaske mit offenen und verschlossenen Bereichen ausgestattet werden.

Durch die Unterteilung von offenen und verschlossenen Bereichen kann ein beabstandeter Klebemittelaustritt aus dem Auftragskopf erhalten werden, was dann beim Vorbeiführen der Teilbahn zu einem beabstandeten Auftrag an adhäsiver Beschichtung in Form von streifenförmigen Kleberzonen führt. Durch die Geometrie der Einlegemaske bzw. der Anordnung der offenen und geschlossenen Bereiche kann das Streifenmuster der Kleberzonen, im Sinne der Breite der Kleberzonen und dem Abstand der Kleberzonen zueinander angepasst und eingestellt werden.

Vorzugsweise ist der Auftragskopf der Beleimungsstation mit Dosiervorrichtungen für das Freisetzen von gewünschten, definiert einstellbare Mengen an Klebemitteln zum Erhalt von vorteilhaften Flächengewichten der adhäsiven Beschichtung ausgestattet.

Die ersten Elastifizierungsmittel werden in einem gedehnten Zustand, vorzugsweise kontinuierlich, der jeweiligen Teilbahn zugeführt. Vorzugsweise werden die ersten Elastifizierungsmittel auf eine Vorspannung von 1,5 - 6,0, vorzugsweise 2,5 - 5,0 gedehnt, vorzugsweise mittels Änderung der Geschwindigkeit im Laufe der Zuführung der Elastifizierungsmittel. Mittels handelsüblichen Fadenführungsvorrichtungen werden die ersten Elastifizierungsmittel in Richtung der Teilbahn ausgerichtet und erfindungsgemäß wird jeweils ein einziges erstes Elastifizierungsmittel innerhalb einer Kleberzone auf der Auftragsseite der Teilbahn positioniert.

Die entsprechend andere, also die zweite oder erste Lage bildende Teilbahn wird mit einer Geschwindigkeit, vorzugsweise der gleichen Geschwindigkeit v1, dem Prozess in Bahnlaufrichtung und auf der Kleberauftragsseite der beschichteten Teilbahn und nach der Positionierung der ersten Elastifizierungsmittel zugeführt, so dass sich die ersten Elastifizierungsmittel im Zwischenraum der die erste und zweite Lage bildenden Teilbahnen befinden. Die Zuführung der anderen Teilbahn erfolgt unmittelbar nach dem Klebstoffauftrag und Positionierung der ersten Elastifizierungsmittel, so dass in direkter Folge innerhalb der Abbindezeit des Klebstoffs die drei Materialien der Laminatbildung zugeführt werden, und dabei insbesondere mittels eines Walzenpaares aufeinander gepresst werden. Durch die in Relation zum Fadendurchmesser D deutlich breiteren Kleberzonen erfolgt so neben der Fixierung des Elastifizierungsmittels im Kleberbett auch eine Verklebung der beiden Teilbahnen zueinander.

Alternativ kann auf diese weitere Teilbahn ebenso eine adhäsive Beschichtung nach dem voranstehend beschriebenen Prozess aufgetragen werden. Hierbei sollte die adhäsive Beschichtung deckungsgleich der bereits beschichteten Teilbahn ausgestaltet sein.

Die Schrittabschnitte sind vorteilhafterweise mit einem Deckblatt-Material, einem Backsheet-Material und einem Absorptionskörper, und einem über den Längsrändern des Absorptionskörper erstreckenden Überhang der Deckblatt- und/oder Backsheet-Materialien, wie voranstehend beschrieben, ausgestattet.

Es wäre grundsätzlich denkbar, dass die zuzuführenden Schrittabschnitte des Inkontinenzartikels derart vorkonfiguriert sind, dass sie endlos, insbesondere von einer Rolle, zugeführt werden. Nach einer weiteren Verfahrensvariante kann es sich als vorteilhaft erweisen, wenn die Schrittabschnitte erst innerhalb des kontinuierlichen Verfahrens gebildet werden, indem eine endlose Topsheet-Materialbahn, eine endlose Backsheet-Materialbahn und Absorptionskörper in einer Maschinenrichtung zugeführt und die Absorptionskörper voneinander beabstandet zwischen die Topsheet-Materialbahn und die Backsheet-Materialbahn angeordnet werden und der so gebildete Verbund in sich fixiert wird.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1    eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem, gespanntem und ausgedehntem Zustand dargestellt sind;

Figur 2    eine Draufsicht auf einen Ausschnitt aus dem Rückenabschnitt aus Figur 1 mit Darstellung von Kleberzonen;

Figur 3    eine Darstellung analog Figur 2 mit alternativer Ausbildung der Kleberzonen;

Figur 4    eine Schnittansicht (schematisch) der Schnittebene II-II des Inkontinenzartikels aus Figur 1;

Figur 5    eine Schnittansicht (schematisch) der Schnittebene III-III des Inkontinenzartikels aus Figur 1;

Figur 6    eine Draufsicht auf einen erfindungsgemäßen Inkontinenzartikel in einer weiteren Ausführungsform, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem, gespanntem und ausgedehntem Zustand dargestellt sind;

| | |
|---|---|
| Figur 7 | eine perspektivische Ansicht (schematisch) eines an einen Benutzer angelegten Inkontinenzartikels in der Ausführungsform nach Figur 6; |
| Figur 8 | eine Draufsicht auf einen Ausschnitt aus dem Bauchabschnitt aus Figur 6; |
| Figuren 9, 10 | verdeutlichen beispielhaft die Bestimmung von Rückstellkräften im Bauchabschnitt bzw. Rückenabschnitt des Inkontinenzartikels; |
| Figuren 11 a, 11 b, 11c | eine schematische Darstellung des erfindungsgemäßen Verfahrens zum Festlegen der ersten Elastifizierungsmittel; |
| Figur 12 | eine schematische Darstellung der Zuführung und Anfügung von Elastifzierungsmittel an den ersten und zweiten Bauchteilabschnitt bzw. Rückenteilabschnitt |
| Figur 13 | eine schematische Darstellung des Faltens auf Höschenform und des Ausbildens von Seitennahtbereichen und anschließender Vereinzelung der Hygieneartikel |

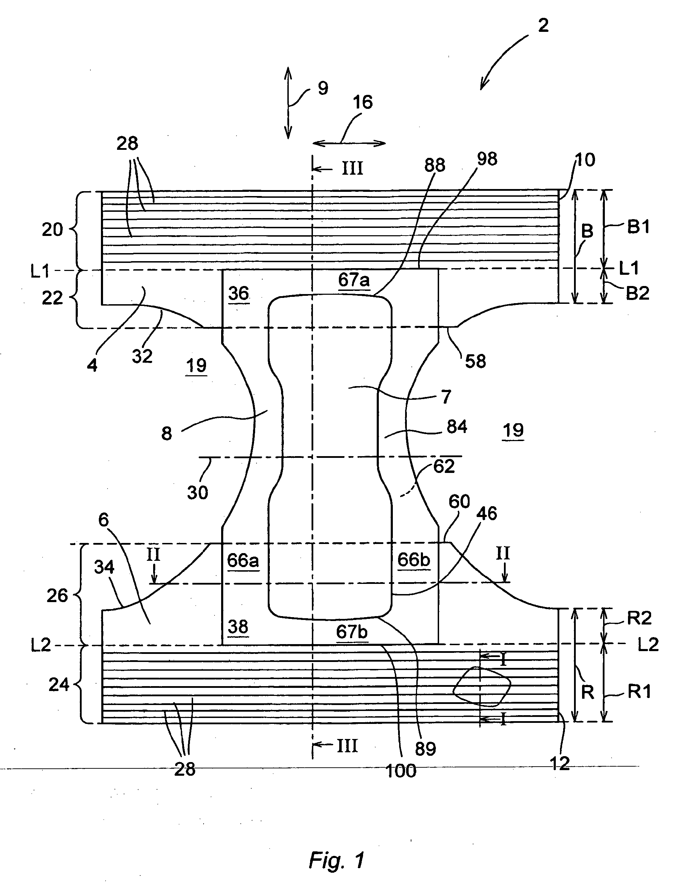

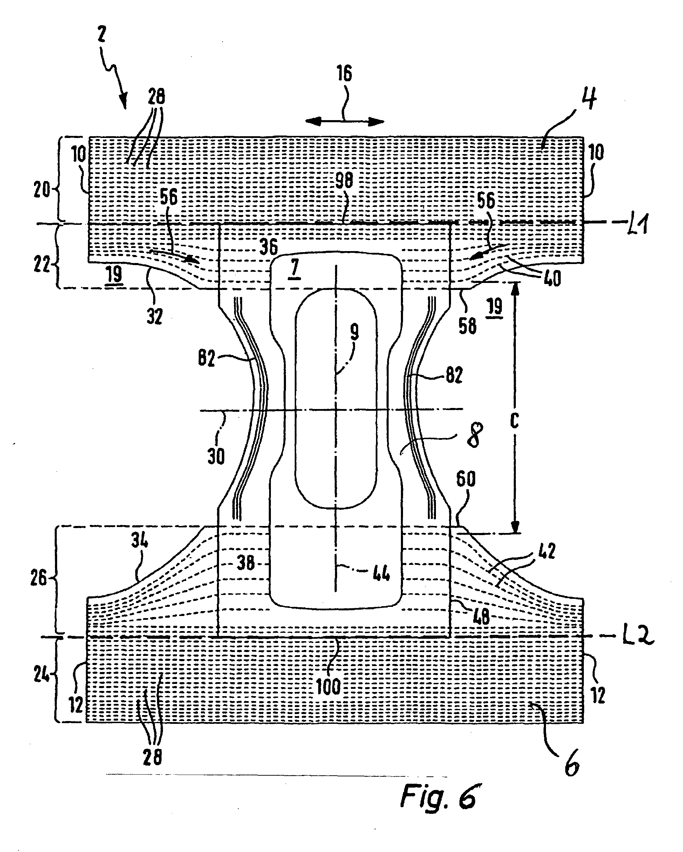

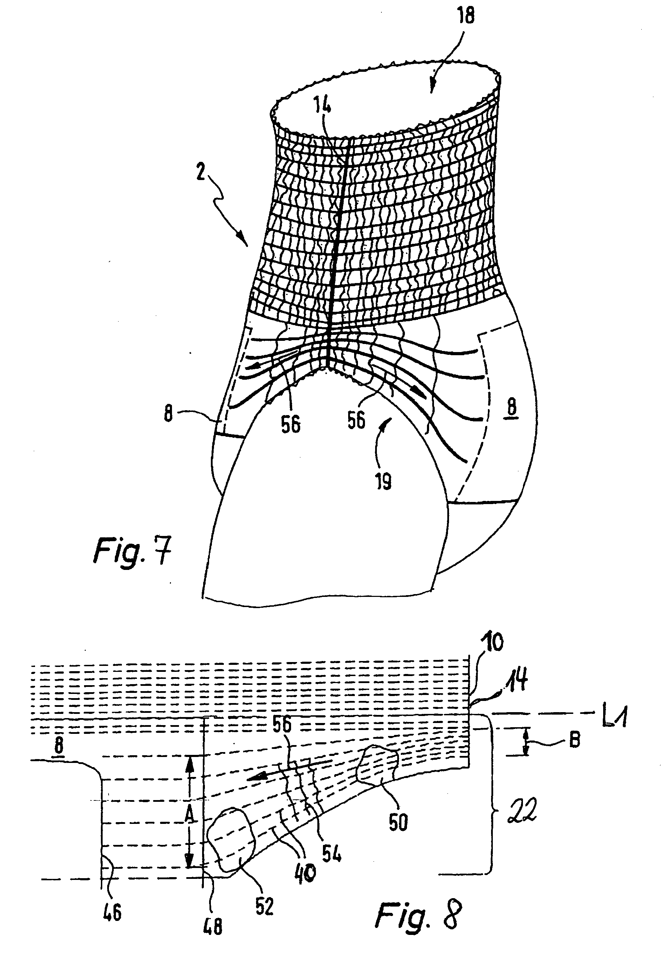

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil mit dem Bauchabschnitt 4 einerseits und dem Rückenabschnitt 6 andererseits überlappt und im Überlappungsbereich 36 von Schrittabschnitt 8 mit Bauchabschnitt 4 und im Überlappungsbereich 38 von Schrittabschnitt 8 mit Rückenabschnitt 6 herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 ausgebildet werden, wie in der schematisch dargestellten Figur 7 zu erkennen ist, wobei die Figur 7 bereits eine weitere Ausführungsform des Inkontinenzartikels in Höschenform (basierend auf Figur 6) zeigt. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann, und zwar auch, wenn kein vollflächiger Kleberauftrag verwendet wird.

Der einen Absorptionskörper 7 aufweisende Schrittabschnitt 8 erstreckt sich in Längsrichtung 9 zwischen dem Bauchabschnitt 4 mit einem schrittzugewandten Querrand 58 und dem Rückenabschnitt 6 mit einem schrittzugewandten Querrand 60 und weist ein bauchseitiges Längsende 98 und ein rückenseitiges Längsende 100 auf.

Das bauchseitige Längsende 98 erstreckt bis zu einer parallel zur Querrichtung 16 verlaufenden gedachten Linie L1 und das rückenseitige Längsende 100 erstreckt bis zu einer parallel zur Querrichtung 16 verlaufenden gedachten Linie L2. Die Linie L1 teilt dabei den Bauchabschnitt 4 in einen ersten beinöffnungsfernen Bauchteilabschnitt 20 und einen zweiten beinöffnungsnahen Bauchteilabschnitt 22 und die Linie L2 teilt den Rückenabschnitt 6 in einen ersten beinöffnungsfernen Rückenteilabschnitt 24 und einen zweiten beinöffnungsnahen Rückenteilabschnitt 26.

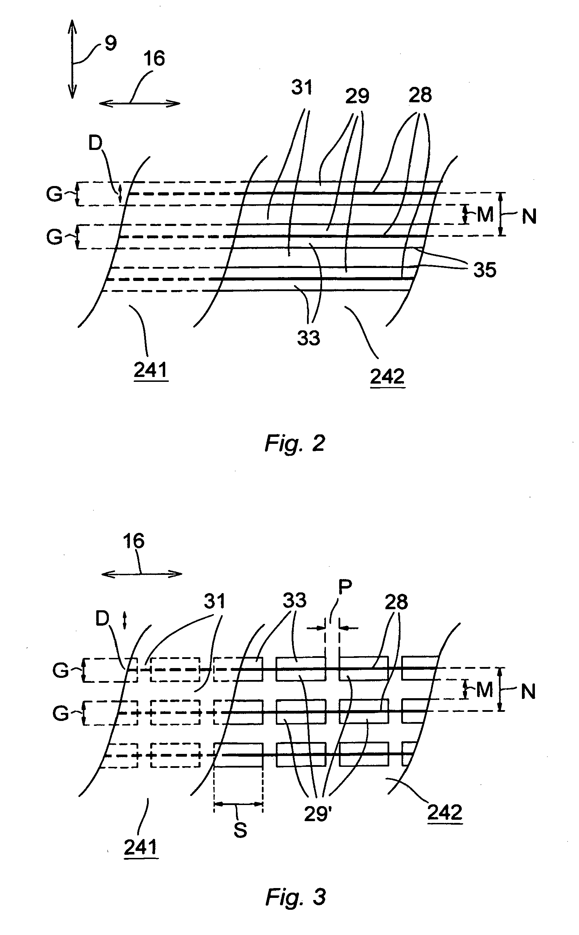

Im ersten beinöffnungsfernen Bauchteilabschnitt 20 und im ersten beinöffnungsfernen Rückenteilabschnitt 24 sind erste Elastifizierungsmittel 28 vorgesehen, bei denen es sich um fadenförmige oder bandförmige Elastifizierungsmittel, insbesondere wie Lycra®- Fäden, handelt, die in vorgedehntem Zustand, im sogenannten Stretch- Bond- Verfahren, mit den Chassismaterialien des Bauchabschnitts 4 und des Rückenabschnitts 6 verbunden sind, wie nachfolgend anhand den Figuren 2 bzw. 3 noch im Detail erläutert wird. Diese ersten Elastifizierungsmittel 28 erstrecken sich in Quer- oder Hüftumfangsrichtung 16 von einem Seitennahtbereich 14 zum anderen.

Die Erstreckung B in Längsrichtung 9 im Seitennahtbereich 14 des Bauchabschnitts 4 und die Erstreckung R in Längsrichtung im Seitennahtbereich 14 des Rückenabschnitts 6 beträgt vorteilhafterweise zwischen 100 und 220 mm.

Die Erstreckung B im Seitennahtbereich setzt sich aus der Erstreckung B1 im Seitennahtbereich des ersten beinöffnungsfernen Bauchteilabschnitts 20 und der Erstreckung B2 im Seitennahtbereich des zweiten beinöffnungsnahen Bauchteilabschnitts 22 zusammen, welche aus der Teilung des Bauchabschnitt 4 durch die gedachte in Querrichtung 16 verlaufende Linie L1 resultieren.

Die Erstreckung in Längsrichtung 9 des ersten beinöffnungsfernen Bauchteilabschnitts 20 im Seitennahtbereich 14 mit einer Länge B1 beträgt dabei vorteilhafterweise wenigstens 60 mm, insbesondere wenigstens 80 mm und insbesondere 80 mm bis 160 mm.

Die Erstreckung R im Seitennahtbereich setzt sich aus der Erstreckung R1 im Seitennahtbereich des ersten beinöffnungsfernen Rückenteilabschnitts 24 und der Erstreckung R2 im Seitennahtbereich des zweiten beinöffnungsnahen Rückenteilabschnitts 26 zusammen, welche aus der Teilung des Rückenabschnitt 6 durch die gedachte in Querrichtung 16 verlaufende Linie L2 resultieren. Die Erstreckung in Längsrichtung 9 des ersten beinöffnungsfernen Rückenteilabschnitts R1 im Seitennahtbereich 14 mit einer Länge R1 beträgt vorteilhafterweise wenigstens 60 mm, insbesondere wenigstens 80 mm, weiter insbesondere 80 mm bis 160 mm.

Die Erstreckung des Schrittabschnitts 8 in Querrichtung 16 beträgt vorteilhafterweise 200 bis 350 mm.

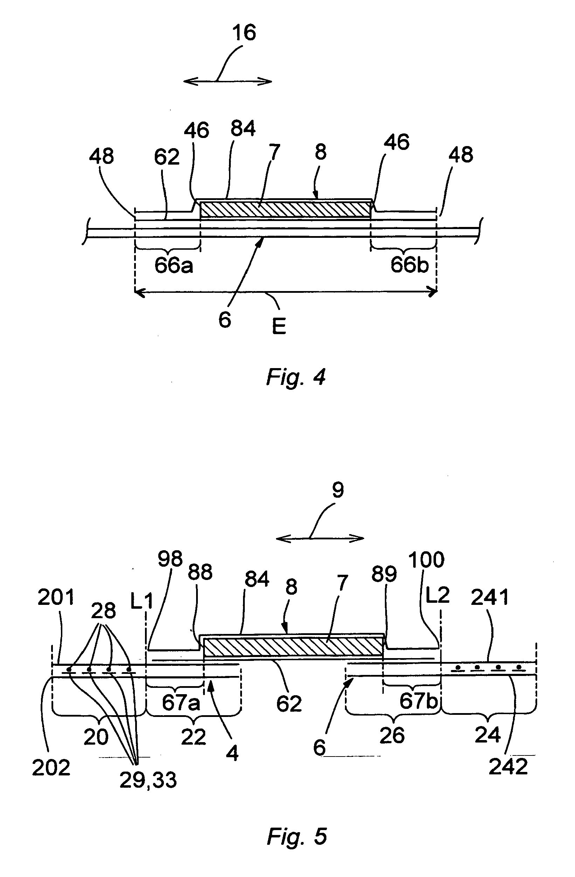

Der Schrittabschnitt 8 umfasst ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis hergestelltes Deckblatt-Material 84. Zwischen dem Backsheet-Material 62 und dem Deckblatt-Material 84 ist der Absorptionskörper 7 angeordnet. Der Absorptionskörper 7 weist Längsränder 46 und einen bauchseitigen Querrand 88 und einen rückenseitigen Querrand 89 auf. Im beispielhaft dargestellten Fall bildet das-Backsheet Material 62 und/oder Deckblarr-Material 84 in Querrichtung 16 beidseits der Längsränder 46 jeweils einen Überhang 66a, 66b. Ebenso bildet das Backsheet-Material 62 und/oder Deckblatt-Material 84 beidseits der Querränder 88, 89 einen Überhang 67a, 67 b aus. Die Figur 4 zeigt dazu schematisch eine Schnittansicht des Inkontinenzartikels aus Figur 1 entlang der in Querrichtung 16 verlaufenden Schnittebene II-II. Der Schrittabschnitt 8 mit einem zwischen dem Deckblatt-Material 84 und dem Backsheet-Material 62 vorhandenen Absorptionskörper 7 überlappt mit dem Rückenabschnitt 6, welcher zumindest zwei Lagen umfasst. Das Deckblatt-Material 84 und das Backsheet-Material 62 erstrecken sich in Querrichtung 16 außerhalb Längsränder 46 des Absorptionskörpers 7 und enden beidseits in den Längsrändern 48 des Schrittabschnitts 8. Deckblatt-Material 84 und Backsheet-Material 46 bilden dabei beidseits einen Überhang 66a, 66b. Vorteilhafterweise beträgt der Überhang des Backsheet-Materials und/oder des Deckblatt-Materials in Querrichtung in der Summe, also beidseits der Längsränder des Absorptionskörpers wenigstens 25%, insbesondere 25 - 50 %, weiter insbesondere 30 - 45 % und weiter insbesondere 35 - 45 % bezogen auf die größte Breite E des Schrittabschnitts. Unter der größten Breite E wird die maximale Erstreckung des Schrittabschnitts 8 in Querrichtung 16 verstanden, welche sich im vorliegenden Fall innerhalb des Überlappungsbereiches von Schrittabschnitt 8 mit Rückenabschnitt 6 befindet. Ein verhältnismäßig großer Überhang ist vorteilhaft, da aufgrund des großen Überlappungsbereichs von Schrittabschnitt und Bauchabschnitt bzw. von Schrittabschnitt und Rückenabschnitt der Schrittabschnitt herstellerseitig sicher an Rücken- und Bauchabschnitt, insbesondere mittels Klebstoffen festlegbar ist.

Des Weiteren ist ein großer Überhang in Querrichtung vorteilhaft bei der Anordnung von Beinelastifizierungsmittel, wie sie beispielhaft in der Ausführungsform nach Figur 6 schematisch dargestellt sind und auch in der Ausführungsform nach Figur 1 in den Schrittabschnitt 8 entlang den Beinöffnungen 19 eingebracht sein können.

Wie in Figur 6 dargestellt, bleibt durch den Überhang 66a, 66b in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das vorteilhaft zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie in Figur 1 schematisch dargestellt, haben der zweite beinöffnungsnahe Bauchteilabschnitt 22 und der zweite beinöffnungsnahe Rückenteilabschnitt 26 eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist bogenförmig und daher zur Begrenzung der Beinöffnungen 19 geeignet. Durch diesen Verlauf des zweiten beinöffnungsnahen Bauchteilabschnitts 22 und des zweiten Rückenteilabschnitts 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Die aus der Figur 1 entlang der in Längsrichtung 9 verlaufenden Schnittebene III - III erhaltende Schnittansicht des Inkontinenzartikels 2 wird in Figur 5 schematisch gezeigt. Der Schrittabschnitt 8 erstreckt sich in Längsrichtung 9 zwischen Bauchabschnitt 4 und Rückenabschnitt 6 und überlappt mit diesen beiden Komponenten. Der Schrittabschnitt 8 weist dabei ein bauchseitiges Längsende 98 und ein rückenseitiges Längsende 100 auf, wobei eine jeweils am Längsende gedachte Linie den Bauchabschnitt und den Rückenabschnitt teilen. Das bauchseitige Längsende 98 erstreckt sich dabei bis zu einer gedachten Linie L1, welche den Bauchabschnitt 4 in einen beinöffnungsfernen ersten Bauchteilabschnitt 20 und in einen beinöffnungsnahen zweiten Bauchteilabschnitt 22 teilt. Das rückenseitige Längsende 100 erstreckt sich dabei bis zu einer gedachten Linie L2, welche den Rückenabschnitt 6 in einen beinöffnungsfernen ersten Rückenabschnitt 24 und in einen beinöffnungsnahen zweiten Rückenteilabschnitt 26 teilt. In dieser Schnittansicht ist auch der Überhang 67a und 67b erkennbar, welcher sich durch die Erstreckung des Deckblatt-Materials 84 und Backsheet- Materials 62 außerhalb des jeweiligen Querrandes 88, 89 des Absorptionskörpers 7 ergibt. Der erste Bauchteilabschnitt 20 und der erste Rückenteilabschnitt 24 weisen jeweils eine erste Lage 201, 241 und eine zweite Lage 202, 242 auf. Zwischen der ersten und zweiten Lage des ersten Bauchteilabschnitts 20 und des ersten Rückenteilabschnitts 24 sind erste Elastifizierungsmittel 28 angeordnet. Die Laminatbildung von jeweils erster Lage, zweiter Lage und Elastifizierungsmittel wird mittels der in den Kleberzonen 29 vorhandenen adhäsiven Beschichtung 33 erreicht. Die Anordnung der Kleberzonen und der ersten Elastifizierungsmittel wird mittels der Figuren 2 und 3 noch näher erläutert.

Figur 2 zeigt schematisch eine vergrößerte Darstellung eines Ausschnitts I-I des ersten beinöffnungsfernen Rückenteilabschnitts 24 nach Figur 1.

Der erste Rückenteilabschnitt 24 umfasst eine erste Lage 241 und eine zweite Lage 242 zwischen denen die ersten Elastifizierungsmittel 28 eingebracht sind. Zur Festlegung der ersten Elastifizierungsmittel 28 sind auf der zweiten Lage 242 streifenförmige Kleberzonen 29 mit einer adhäsiven Beschichtung 33 aufgebracht. Die Kleberzonen verlaufen dabei in Querrichtung 16 des ersten Rückenteilabschnitts und sind in Längsrichtung 9 parallel mit einem Abstand M beabstandet angeordnet. Die streifenförmigen Kleberzonen 29, begrenzt durch die in Querrichtung 16 verlaufenden Kanten 35, weisen eine Breite G auf. Der Abstand M zwischen den streifenförmigen Kleberzonen 29 bemisst sich dabei über die Entfernung der in Querrichtung 16 benachbart verlaufenden Kanten 35 von direkt benachbarten Kleberzonen 29. Zwischen den streifenförmigen Kleberzonen 29 verbleibt eine kleberzonenfreie Fläche 31. Innerhalb einer streifenförmigen Kleberzone 29 wird nur jeweils ein einziges fadenförmiges bzw. bandförmiges erstes Elastifizierungsmittel 28 mit einem Fadendurchmesser D eingebracht, wobei der Fadendurchmesser D stets geringer ist als die Breite G der streifenförmigen Kleberzone 29. Der Abstand N der ersten Elastifizierungsmittel 28 (gemessen als Entfernung zwischen direkt benachbarten ersten Elastifizierungsmittel 28) ist damit auch stets größer als der Abstand M zwischen den jeweiligen benachbarten streifenförmigen Kleberzonen 29.

Es ist auch denkbar, dass anstelle der ersten Lage 241 die zweite Lage 242 Kleberzonen mit adhäsiver Beschichtung aufweist (nicht dargestellt). Ebenso ist alternativ möglich, dass sowohl die erste Lage 241 als auch die zweite Lage 242 Kleberzonen mit adhäsiver Beschichtung aufweisen (nicht dargestellt). Im letzteren Fall ist es vorteilhaft, wenn die erste Lage und die zweite Lage ein deckungsgleiches Auftragsmuster an Kleberzonen aufweisen, welche dann bei der Laminatbildung insbesondere bevorzugt übereinstimmend übereinander angeordnet werden.

Figur 3, ebenso eine vergrößerte und schematisch detaillierte Darstellung des Ausschnittes I - I aus der Figur 1 zeigt analog zu Figur 2 zwischen einer ersten und einer zweiten Lage 241, 242 innerhalb von Kleberzonen einzeln eingebrachte erste Elastifizierungsmittel 28, aber in einer alternativen Ausführungsform, derart, dass die streifenförmigen Kleberzonen 29 in Querrichtung 16 in einem Abstand P angeordnete Kleberzonenabschnitten 29' umfassen. Die Kleberzonenabschnitte 29' weisen eine Länge S auf, welche vorteilhafterweise in ihren Abmessungen in Querrichtung 16 größer ist als der Abstand P. Insbesondere beträgt der Quotient S/P mindestens 0,1, aber vorzugsweise höchstens 15. Die Kleberzonenabschnitte weisen vorteilhaft eine Länge S von mindestens 2 mm, und höchstens 30 mm auf. Der Abstand P der direkt benachbarten Kleberzonenabschnitte beträgt insbesondere mindestens 2 mm, weiter insbesondere höchstens 20 mm.

Die Kleberzonen 29 bzw. die Kleberzonenabschnitte 29' weisen eine Breite G auf und sind in Längsrichtung in einem Abstand M parallel angeordnet. Vorteilhaft sind die Breite G der Kleberzonen und der Abstand M aufeinander abgestimmt. Der Quotient G/M beträgt vorteilhafterweise mindestens 0,10 und vorzugsweise höchstens 7,5.

Die Kleberzonen 29 bzw. die Kleberzonenabschnitte 29' weisen vorteilhaft eine Breite G zwischen 2 und 15 mm auf und sind vorteilhaft in einem Abstand M zwischen 2 und 25 mm parallel angeordnet. Die ersten Elastifizierungsmittel 28 weisen einen im Vergleich zur Breite G der Kleberzonen bzw. Kleberzonenabschnitte geringeren Fadendurchmesser D auf, vorteilhafterweise derart, dass der Quotient G/D mindestens 2 und höchstens 100 beträgt. Insbesondere vorteilhaft werden Elastifizierungsmittel mit einem Fadendurchmesser D von 0,05 - 1,0 mm eingesetzt. Die adhäsive Beschichtung 33 innerhalb der Kleberzonen 29 ist dabei in Form eines Klebers, insbesondere eines Hotmeltklebers aufgebracht. Besonders bevorzugt ist hierbei ein hydrophober Kleber, insbesondere der Kleber LC 3001ZP von der Firma Fuller (H.B. Fuller Deutschland GmbH, An der Roten Bleiche 2-3, 21335 Lüneburg, Deutschland) aufgebracht. Die adhäsive Beschichtung 33 ist dabei in einem Flächengewicht von 2-40 g/m$^2$ aufgebracht.

Figur 6 zeigt eine Draufsicht auf einen erfindungsgemäßen Inkontinenzartikel in einem noch ausgebreiteten und in einem noch nicht in den Längsrandabschnitten von Bauchabschnitt und Rückenabschnitt verbundenen Zustand, wobei zusätzlich zu den ersten Elastifizierungsmittel 28 im ersten Bauchteilabschnitt 20 und im ersten Rückenteilabschnitt 24 der Inkontinenzartikel 2 auch im zweiten beinöffnungsnahen Bauchteilabschnitt 22 und im zweiten beinöffnungsnahen Rückenteilabschnitt 26 elastifiziert ausgebildet ist. Die Bezugszeichen wie in Figur 1 erläutert sind auf die Figur 6 zu heranzuziehen.

Im zweiten beinöffnungsnahen Bauchteilabschnitt 22 und Rückenteilabschnitt 26 sind zweite Elastifizierungsmittel 40 bzw. 42 vorgesehen. Die zweiten Elastifizierungsmittel 40, 42 erstrecken sich jeweils ausgehend von den Längsrandabschnitten 10, 12 von Bauchabschnitt 4 bzw. Rückenabschnitt 6 in Richtung auf eine Längsmittelachse 44 des Inkontinenzartikels. Wie aus der Figur 6 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 8 auch quantitativ näher bezeichnen. Beispielsweise haben die in Figur 8 dargestellten zweiten Elastifizierungsmittel 40 des zweiten beinöffnungsnahen Bauchabteilschnitts 22 in den Längsrandabschnitten 10 bzw. Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 8 auch ein Auffächerungsgrad F wie folgt definieren:

$$F = (A-B)/B * 100\%$$

Dieser Auffächerungsgrad kann vorteilhafterweise zwischen 50 und 900%, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden Elastifizierungsmitteln), und zwar A als der maximale Abstand, insbesondere am Längsrand 48 des Schrittabschnitts 8, und B als der minimale Abstand, insbesondere im Seitennahtbereich 14 (vgl. Figur 8).

Sofern der Auffächerungsgrad bei den zweiten Elastifizierungsmitteln 40, 42 hinreichend groß gewählt wird, so lässt sich auf diese Weise eine abnehmende Rückstellkraft innerhalb des zweiten beinöffnungsnahen Bauchteilabschnitts 22 beziehungsweise des zweiten beinöffnungsnahen Rückenteilabschnitts 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden zweiten beinöffnungsnahen Bauchteilabschnitts 22 beziehungsweise Rückenteilabschnitts 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des zweiten beinöffnungsnahen Bauchteilabschnitts 22 beziehungsweise des zweiten beinöffnungsnahen Rückenteilabschnitts 26 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine bewusst vorteilhaft vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen.

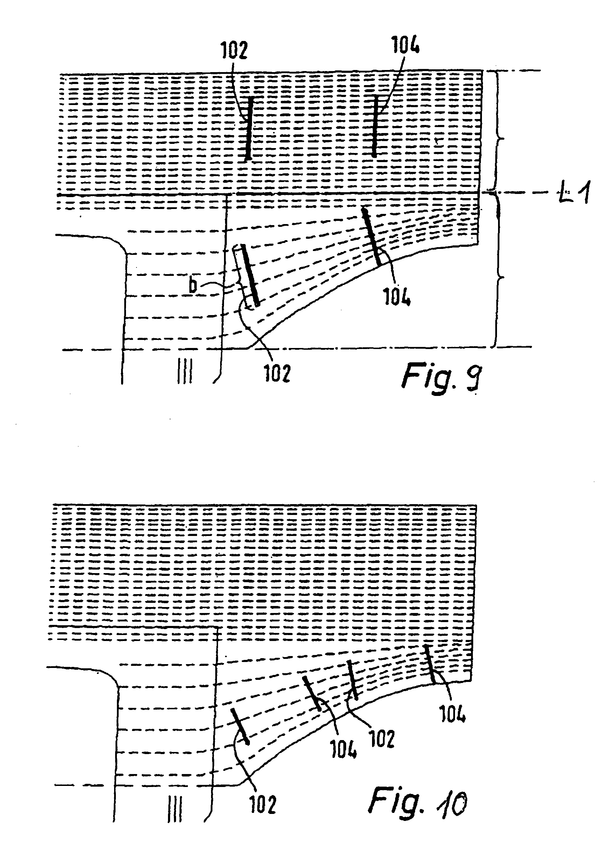

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts zwischen zwei Klemmbacken 102, 104 (siehe Fig. 9, 10) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstands im ungespannten Zustand) ermittelt. Die Klemmbacken 102, 104 werden dabei jeweils voneinander wegbewegt. Die Klemmbacken 102, 104 sollten möglichst viele, zumindest jedoch zwei nebeneinander angeordnete erste bzw. zweite Elastifizierungsmittel 28, 40,42 des zu messenden Bereichs fixieren, und sie sollten im Wesentlichen rechtwinklig zum Verlauf der Elastifzierungsmittel orientiert sein, so das die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figuren 9 und 10 verwirklicht.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 voneinander beträgt 250-400 mm.

Der Abstand der innersten, schrittzugewandten zweiten Elastifizierungsmittel 40, 42 von der die Beinöffnungen begrenzenden Randkontur 32, 34 des zweiten beinöffnungsnahen Bauchteilabschnitts 22 und Rückenteilabschnitts 26 beträgt vorzugsweise 2-40 mm, weiter vorzugsweise 3-30 mm, insbesondere vorzugsweise 4-15 mm.

Erfindungsgemäßes Verfahren:

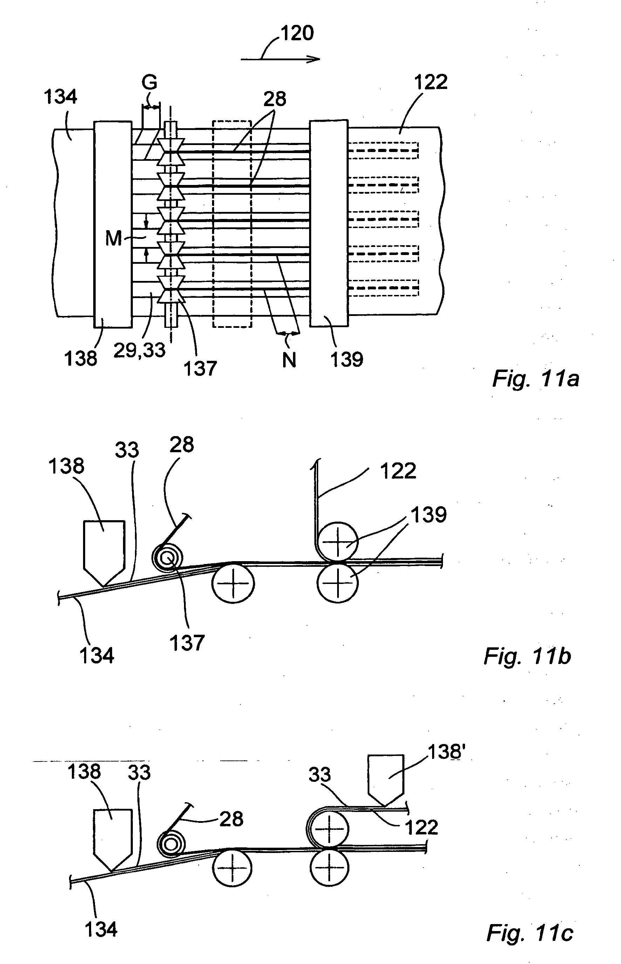

Die Figuren 11 bis 13 zeigen das erfindungsgemäße Herstellungsverfahren:

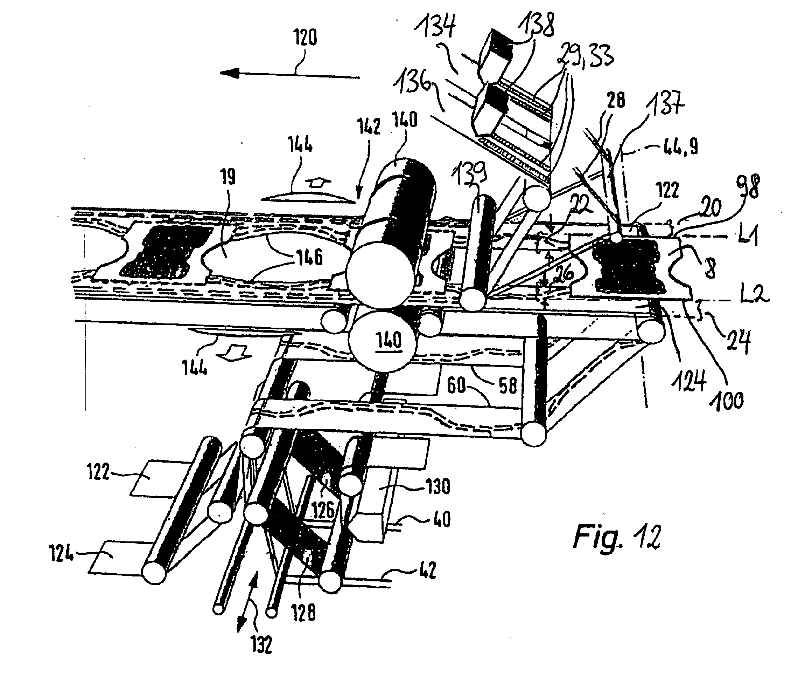

Wie aus Figur 12 ersichtlich ist, werden zur Herstellung des späteren Bauchabschnitts 4 und Rückenabschnitts 6 des Inkontinenzartikels 2, so wie in den Figuren 1 oder 4 in einem noch ausgebreiteten und nicht an den Längsrandabschnitten von Bauchabschnitt und Rückenabschnitt verbundenen Zustand dargestellt, Teilbahnen 122, 124, vorzugsweise auf Vliesbasis, zugeführt. Diese Teilbahnen 122, 124 können ausgehend von einer einzigen Bahn durch Trennen in Längsrichtung gebildet sein.

Diese Teilbahnen 122, 124 werden mit Schrittabschnitten 8 zusammengeführt, und zwar derart, dass die Schrittabschnitte in einer Längsrichtung 9 quer zur Maschinenrichtung 120 einenends mit der einen Teilbahn 122 und anderenends mit der anderen Teilbahn 124 überlappen. Der Schrittabschnitt 8 weist dabei ein bauchseitiges Längsende 98 und ein rückenseitiges Längsende 100 auf. Diese Längsenden 98, 100 erstrecken sich dabei bis zu einer parallel verlaufenden gedachten Linie L1 bzw. L2. Die gedachte Linie L1 teilt die den späteren Bauchabschnitt bildende Teilbahn 122 in einen beinöffnungsfernen ersten Bauchteilabschnitt 20 und einen beinöffnungsnahen zweiten Bauchteilabschnitt 22. Analog teilt die gedachte Linie L2 die den späteren Rückenabschnitt bildende Teilbahn 124 in einen beinöffnungsfernen ersten Rückenteilabschnitt 24 und einen beinöffnungsnahen zweiten Rückenteilabschnitt 26.

Es werden weitere Teilbahnen 134, 136, insbesondere auf Vliesbasis, zugeführt, welche ebenso für die Bildung des späteren Bauchabschnitt und Rückenabschnitt vorgesehen sind.

Weiterhin werden dann die in Quer- oder Hüftumfangsrichtung 16 erstreckten für die Festlegung im späteren ersten Bauchteilabschnitt und im späteren ersten Rückenteilabschnitt vorgesehenen ersten Elastifizierungsmittel 28 endlos in der Maschinenrichtung 120 zugeführt. Insbesondere werden die ersten Elastifizierungsmittel um Fadenführungsvorrichtungen 137 beabstandet voneinander und unter Erhalt einer Vorspannung zugeführt. Für die Fixierung der ersten Elastifizierungsmittel 28 zwischen den jeweils zusammen wirkenden Teilbahnen 122, 134 zur Bildung des Bauchabschnitts, und damit des ersten Bauchteilabschnitts 20 und zwischen den Teilbahnen 124, 136 zur Bildung des Rük-kenabschnitt und damit des ersten Rückenteilabschnitts 24, werden die Teilbahnen mit einer adhäsiven Beschichtung 33 versehen. Die adhäsive Beschichtung 33 wird dabei in Form von streifenförmigen Kleberzonen 29 auf die Teilbahnen aufgebracht. Im vorgestellten Ausführungsbeispiel wird die adhäsive Beschichtung 33 auf nur jeweils eine der beiden Teilbahnen, also auf die erste oder zweite Lage des späteren ersten Bauchteilabschnitts, hier 134 und auf nur jeweils eine der beiden Teilbahnen, also auf die erste oder zweite Lage des späteren ersten Rückenteilabschnitts, hier 136, aufgebracht. Es sind aber auch die  Alternativen des Auftrags der adhäsiven Beschichtung auf die jeweils andere der Teilbahnen oder auch auf beide Teilbahnen denkbar, wie später noch anhand Figur 11a-c schematisch skizziert dargelegt wird.

Zum Auftrag der adhäsiven Beschichtung 33 in streifenförmigen in Maschinenrichtung 120 parallel und beabstandet angeordneten Kleberzonen 29 werden die Teilbahnen 134, 136 an einer Beleimungsstation 138 vorbeigeführt, so dass der Kleberauftrag insbesondere im Kontaktverfahren erfolgt.

Die mit Kleberauftrag versehenen Teilbahnen und die ersten Elastifizierungsmittel werden zur Laminatbildung zusammengeführt, derart, dass jeweils nur ein einziges erstes Elastifizierungsmittel 28 innerhalb einer Kleberzone 29 entlang der Maschinenrichtung 120 zu liegen kommt. Dem nachfolgend werden die Materialien, also die jeweiligen Teilbahnen und die Elastifizierungsmittel innerhalb der Abbindezeit des Klebstoffes 33 mittels einer Walze 139, insbesondere eines Walzenpaares 139 aufeinander gepresst.

In den Figuren 11a, b, c wird das Verfahren zur Fixierung von gedehnten, also mit einer Vorspannung versehenen ersten Elastifizierungsmittel nochmals veranschaulicht. Fig. 11 a zeigt dabei schematisch eine Draufsicht auf den Laminierungsschritt anhand des späteren ersten Bauchteilabschnitts und damit die eine erste Lage 201 und die eine zweite Lage 202 bildenden Teilbahnen 122, 134.

Von der Beleimungsstation 138 wird auf die darunter vorbeigeführte Teilbahn 134 Klebemittel als adhäsive Beschichtung 33 abgegeben. Der Auftragskopf der Beleimungsstation verfügt über voneinander beabstandete Öffnungen (hier nicht dargestellt). Unter Vorbeiführung der Teilbahn 134 in Maschinenrichtung 120 mit einer definierten Geschwindigkeit werden streifenförmige und parallel in einem Abstand M voneinander angeordnete Kleberzonen 29 mit einer Breite G erzeugt. Über eine Fadenführungsvorrichtung 137 werden die ersten Elastifizierungsmittel 28 vereinzelt in einem Abstand N im gedehnten Zustand oberhalb den mit adhäsiver Beschichtung 33 versehenen Kleberzonen 29 positioniert. Unter Zuführung einer weiteren Teilbahn 122 werden alle Materialien mittels einer Walze 139 zusammengepresst. Die Figuren 11b und 11 c zeigen den vorangehend erläuterten Vorgang schematisch in der Seitenansicht, wobei die Figur 11c in Abwandlung dazu die Alternative eines Kleberauftrags auf beiden Teilbahnen 134, 122 skizziert darstellt. Hierbei wird die Teilbahn 122 mit der der Teilbahn 134 zugewandten Oberseite ebenso unter eine Beleimungsstation 138' zum Auftragen einer adhäsiven Beschichtung 33 vorbeigeführt und dann mit der Teilbahn 134 und den ersten Elastifizierungsmittel 28 zusammengeführt. Vorteilhafterweise wird die Teilbahn 122 mit einer adhäsiven Beschichtung 33 im mit dem der Teilbahn 134 deckungsgleichen Auftragsmuster versehen, so dass dann die ersten Elastifizierungsmittel zwischen der adhäsiven Beschichtung 33 der Teilbahn 134 und der Teilbahn 122 zu liegen kommen und damit fixiert werden.

In der Figur 12 ist weiterhin das Einbringen von zweiten Elastifizierungsmittel 40, 42 zum Erhalt eines weiteren elastifizierten Bereichs des späteren Bauchabschnitts 4 und Rückenabschnitts 6 des Inkontinenzartikels 2, so wie in der Figur 4 in einem noch ausgebreiteten und nicht an den Längsrandabschnitten von Bauchabschnitt und Rückenabschnitt verbundenen Zustand dargestellt ist.

Auf diese eingangs erwähnten Teilbahnen 122, 124 werden die zweiten Elastifizierungsmittel 40 und 42 aufgebracht, die hierfür ebenfalls endlos und in Förderrichtung der Teilbahnen 122, 124 zugeführt werden. Zur Fixierung der zweiten Elastifizierungsmittel 40, 42 auf den Teilbahnen 122, 124 wird jeweils eine Vliesdeckschicht 126, 128, die zuvor in einer Beleimungsstation 130 mit Kleber vorzugsweise vollflächig beaufschlagt wurde, aufgebracht, so dass die zweiten Elastifizierungsmittel 40, 42 zwischen den Teilbahnen 122, 124 und den Vliesdeckschichten 126, 128 einlaminiert werden. Obschon aus Figur 12 infolge der schematischen Darstellung nicht ersichtlich, werden die zweiten Elastifizierungsmittel 40, 42 in einem variierenden Abstand voneinander zugeführt, was durch eine oszillierende Führungseinrichtung, die durch den Doppelpfeil 132 angedeutet ist, realisiert wird. So wird durch entsprechende Ansteuerung der Führungseinrichtung für jedes einzelne der Elastifizierungsmittel 40, 42 der bogenförmige sich auffächernde Verlauf der zweiten Elastifizierungsmittel 40, 42 in Richtung auf den Schrittabschnitt 8 gebildet.

Die zweiten Elastifizierungsmittel 40, 42 werden dabei so zugeführt, dass sie auf den Teilbahnen innerhalb des späteren zweiten Bauchteilabschnitts 22 und des späteren zweiten Rückenteilabschnitts 26 zu liegen kommen.

Es wäre aber möglicherweise auch denkbar, dass die Elastifizierungsmittel 40 und 42 alle einzeln mit Kleber versehen werden, also fadenbeleimt werden.

Es wäre möglicherweise auch denkbar, dass unabhängig von der Art des Klebemittelauftrags auf die Elastifizierungsmittel 40 und 42 auf die Vliesdeckschichten 126 und/oder 128 verzichtet werden kann. Allerdings haben die Vliesdeckschichten den Vorteil, dass sie zugleich eine weich anfühlende Innenseite des Inkontinenzartikels bilden.

Auch in der Ausführungsform mit den zweiten Elastifizierungmittel 40, 42 werden die Schrittabschnitte 8 so zugeführt, dass sie nach dem Zusammenführen mit einem Abstand in der Maschinenrichtung 120 voneinander angeordnet sind. Die Schrittabschnitte 8 und die Teilbahnen 122, 124 werden in der aus Figur 12 erhaltenen Konfiguration miteinander fixiert und in der Maschinenrichtung 120 weitergefördert und mit den ersten Elastifizierungsmittel 28, so wie bereits voranstehend beschrieben, ausgestattet.

Dem nachfolgend ist in Figur 12 ein Schneidwalzenpaar 140, also ein Rotationsmesser mit einer Ambosswalze, vorgesehen, zwischen denen der bislang gebildete Verbund in der Maschinenrichtung 120 in der beschriebenen Orientierung hindurchgeführt wird. Dabei wird ein Konturschnitt 142 ausgeführt, im Zuge dessen, vorzugsweise nur von den Teilbahnen 122, 124, je ein bogenförmiges Segment 144 abgetrennt wird, und zwar von den einander zugewandten Querrändern oder Randabschnitten 58 und 60 der Teilbahnen 122, 124, so dass Beinausschnitte 146 auch bei den Teilbahnen 122, 124 gebildet werden. Dadurch, dass der Konturschnitt 142 nicht den Schrittabschnitt 8 sondern nur die Teilbahnen 122, 124 erfasst, verläuft der Konturschnitt 142 im Wesentlichen entlang der Maschinenrichtung 120 und jedenfalls nicht unter großem Winkel quer hierzu. Auf diese Weise kann der Schnitt optimal konfiguriert werden. Insgesamt können so die späteren Beinöffnungen 19 des Inkontinenzartikels 2 mit hoher Genauigkeit den als optimal erachteten Anforderungen entsprechend ausgebildet werden. Dabei erweist es sich als vorteilhaft, dass der Konturschnitt 142 an der Teilbahn 122 mit einem anderen Verlauf ausgebildet sein kann als an der Teilbahn 124. So kann die Gestalt der Beinausschnitte 146 bzw. der späteren Beinöffnungen 19 des Inkontinenzartikels 2 im Bauchabschnitt 4 und im Rückenabschnitt 6 verschieden konfiguriert werden.

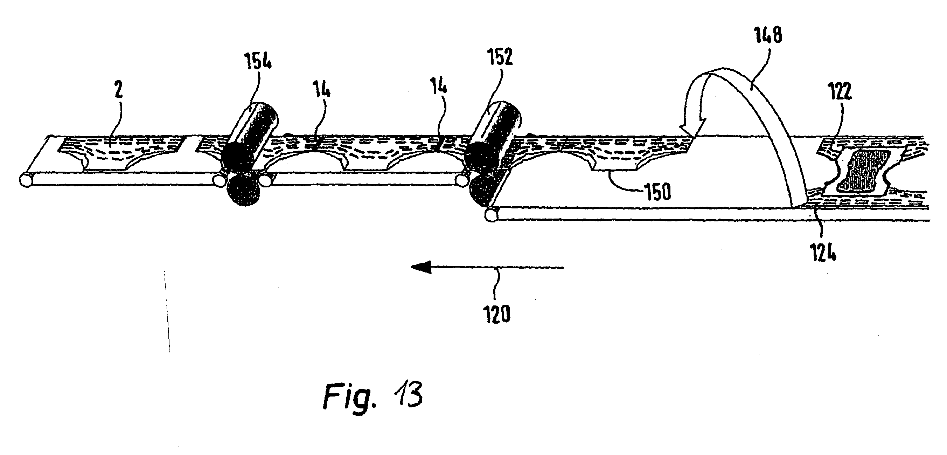

Der so gebildete Verbund wird weitergefördert, und in einer in Figur 13 nur angedeuteten Faltstation 148 wird der Verbund um eine in Maschinenrichtung 120 verlaufende Faltlinie 150 auf sich selbst gefaltet, derart, dass die eine Teilbahn 124 über die andere Teilbahn 122 zu liegen kommt. Daran anschließend wird in einer Fügestation 152 ein jeweiliger Seitennahtbereich 14 zwischen den Teilbahnen 122, 124 ausgebildet, es wird also die eigentliche Höschenform gebildet. An diesen Verfahrensschritt anschließend wird in einer Trennstation 154 ein Trennschnitt quer zur Maschinenrichtung 120 ausgeführt, der zur Vereinzelung der fertigen Inkontinenzartikel 2 führt. Es wäre auch denkbar, dass die Fügestation 152 zugleich als Trennstation ausgebildet ist, beispielsweise in Form einer Trennschweißeinrichtung, so dass zusammen mit der Ausbildung des Seitennahtbereichs 14 die Inkontinenzartikel 2 vereinzelt werden.

Die in Figur 13 skizzierten Verfahrensschritte sind auch auf die Ausführungsform des Inkontinenzartikels mit nur ersten Elastifizierungsmittel, also ohne zweite Elastizierungsmittel, anzuwenden.

Es wäre grundsätzlich denkbar, dass die zuzuführenden Schrittabschnitte 8 des Inkontinenzartikels derart vorkonfiguriert sind, dass sie endlos, insbesondere von einer Rolle, zugeführt werden. Nach einer weiteren Verfahrensvariante kann es sich als vorteilhaft erweisen, wenn die Schrittabschnitte erst innerhalb des kontinuierlichen Verfahrens gebildet werden, indem eine endlose Topsheet-Materialbahn, eine endlose Backsheet-Materialbahn und Absorptionskörper in einer Maschinenrichtung zugeführt und die Absorptionskörper voneinander beabstandet zwischen die Topsheet-Materialbahn und die Backsheet-Materialbahn angeordnet werden und der so gebildete Verbund in sich fixiert wird.

## Patentansprüche

1. Inkontinenzartikel (2) in Höschenform für die Aufnahme von Körperausscheidungen mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), wobei der Schrittabschnitt mit einem bauchseitigen Längsende (98) und einem rückenseitigen Längsende (100) sich in einer Längsrichtung (9) zwischen dem Bauchabschnitt (4) mit einem schrittzugewandten Querrand (58) und dem Rückenabschnitt (6) mit einem schrittzugewandten Querrand (60) erstreckt, und wobei sich das bauchseitige Längsende (98) in Längsrichtung (9) bis zu einer parallel zur Querrichtung (16) verlaufenden gedachten Linie L1 erstreckt, und wobei sich das rückenseitige Längsende (100) in Längsrichtung (9) bis zu einer parallel zur Querrichtung (16) verlaufenden gedachten Linie L2 erstreckt, und wobei der Schrittabschnitt (8) in einem vorderen Überlappungsbereich (36) den Bauchabschnitt (4) überlappt und an den Bauchabschnitt unlösbar angefügt ist und wobei der Schrittabschnitt (8) in einem hinteren Überlappungsbereich (38) den Rückenabschnitt (6) überlappt und an den Rückenabschnitt (6) unlösbar angefügt ist, wobei der Schrittabschnitt (8) ein flüssigkeitsundurchlässiges Backsheet-Material (62) und ein Deckblatt-Material (84) umfasst, und wobei der Absorptionskörper (7) zwischen Backsheet-Material (62) und Deckblatt-Material (84) angeordnet ist, wobei das Deckblatt-Material (84) und/oder das Backsheet-Material (62) in Querrichtung (16) einen sich jeweils außerhalb von Längsrändern (46) des Absorptionskörpers (7) erstreckenden Überhang (66a, 66b) bilden, wobei sowohl der Schrittabschnitt (8) als auch der Bauchabschnitt (4) und der Rückenabschnitt (6) die Beinöffnungen (19) des Inkontinenzartikels begrenzen, wobei die Linie L1 den Bauchabschnitt (4) in einen ersten beinöffnungsfernen Bauchteilabschnitt (20) und einen zweiten beinöffnungsnahen Bauchteilabschnitt (22) und die Linie L2 den Rückenabschnitt (6) in einen ersten beinöffnungsfernen Rückenteilabschnitt (24) und einen zweiten beinöffnungsnahen Rückenteilabschnitt (26) teilen, wobei in dem ersten Bauchteilabschnitt (20) und dem ersten Rückenteilabschnitt (24) erste fadenförmige- oder bandförmige Elastifizierungsmittel (28) mit einem Fadendurchmesser D vorgesehen sind, die sich in einem Abstand N voneinander und parallel zueinander in Quer- oder Hüftumfangsrichtung (16) erstrecken und so den ersten Bauchteilabschnitt (20) und den ersten Rückenteilabschnitt (24) flächenhaft elastifizieren, wobei der erste Bauchteilabschnitt (20) und der erste Rückenteilabschnitt (24) jeweils mindestens eine erste Lage (201, 241) und eine zweite Lage (202, 242) aufweisen, zwischen denen die ersten Elastifizierungsmittel (28) mittels streifenförmiger Kleberzonen (29) festgelegt sind, wobei die streifenförmigen Kleberzonen in Querrichtung- oder Hüftumfangsrichtung (16) mit einer Breite G parallel und in einem Abstand M zueinander angeordnet sind, wobei die Breite G der streifenförmigen Kleberzonen (29) größer ist als der Fadendurchmesser D der ersten Elastifizierungsmittel (28), und wobei jeweils ein einziges erstes Elastifizierungsmittel (28) innerhalb einer jeweiligen streifenförmigen Kleberzone (29) angeordnet ist.

2. Inkontinenzartikel nach Anspruch 1, **dadurch gekennzeichnet, dass** der Quotient G/M mindestens 0,10, insbesondere mindestens 0,30, weiter insbesondere mindestens 0,50, weiter insbesondere mindestens 0,75, weiter insbesondere mindestens 1,0 , vorzugsweise höchstens 7,5, weiter vorzugsweise höchstens 6,0, weiter vorzugsweise höchstens 5,0 , weiter vorzugsweise höchstens 4,0 beträgt.

3. Inkontinenzartikel nach Anspruch 1 oder 2, **dadurch gekennzeichnet, dass** der Quotient G/D mindestens 2, weiter vorzugsweise mindestens 4, weiter vorzugsweise mindestens 6, weiter vorzugsweise mindestens 8, weiter vorzugsweise mindestens 10, vorzugsweise aber höchstens 100, weiter vorzugsweise höchstens 80, weiter vorzugsweise höchstens 60, weiter vorzugsweise höchstens 50, weiter vorzugsweise höchstens 40, weiter vorzugsweise höchstens 30 beträgt.

4. Inkontinenzartikel nach einem oder mehreren der vorstehenden Ansprüche, **dadurch gekennzeichnet, dass** die streifenförmigen Kleberzonen (29) aus in Querrichtung (16) in einem Abstand P angeordneten Kleberzonenabschnitten (29') mit einer Länge S umfassen, welche vorzugsweise derart angeordnet sind, dass der Quotient S/P mindestens 0,1, weiter vorzugsweise mindestens 0,3, weiter vorzugsweise mindestens 0,5, weiter vorzugsweise mindestens 1,0 , weiter vorzugsweise mindestens 2,0, aber weiter vorzugsweise höchstens 15, weiter vorzugsweise höchstens 13, weiter vorzugsweise höchstens 10 beträgt.

5. Inkontinenzartikel nach einem oder mehreren der vorstehenden Ansprüche, **dadurch gekennzeichnet, dass** der erste beinöffnungsferne Bauchteilabschnitt (22) und/oder der erste beinöffnungsferne Rückenteilabschnitt (24) einen Anteil von 30 - 75%, weiter insbesondere von 35-70 %, weiter insbesondere von 35 - 65 % bezogen auf die Fläche des Bauchabschnitts (4) und/oder des Rückenabschnitts (6) einnimmt.

6. Inkontinenzartikel nach einem oder mehreren der vorstehenden Ansprüche, **dadurch gekennzeichnet, dass** der erste beinöffnungsferne Bauchteilabschnitt (22) und/oder der erste beinöffnungsferne Rückenteilabschnitt (24) eine von Kleberzonen (29) und/oder von Kleberzonenabschnitte (29') freie Fläche (31) mit einem Anteil von 20 - 80 %, insbesondere von 25 - 70 % , weiter insbesondere von 30 - 60 % bezogen auf die Fläche des ersten beinöffnungsfernen Bauchteilabschnitts (22) und/oder des ersten beinöffnungsfernen Rückenteilabschnitts (24) aufweist.

7. Inkontinenzartikel nach einem oder mehreren der vorstehenden Ansprüche, **dadurch gekennzeichnet, dass** die Kleberzonen und/oder Kleberzonenabschnitte (29, 29') eine adhäsive Beschichtung (33) mit einem Flächengewicht von 2- 40 g/m$^2$, weiter insbesondere von 2-30 g/m$^2$, weiter insbesondere von 2-20 g/m$^2$, weiter insbesondere von 2-10 g/m$^2$ aufweisen.

8. Inkontinenzartikel nach einem oder mehreren der vorstehenden Ansprüche, **dadurch gekennzeichnet, dass** die adhäsive Beschichtung (33) auf der ersten und/oder zweiten Lage des ersten Bauchteilabschnitts (22) und auf der ersten und/oder zweiten Lage des ersten Rückenteilabschnitts (24) aufgebracht ist.

9. Inkontinenzartikel nach einem oder mehreren der vorstehenden Ansprüche, **dadurch gekennzeichnet, dass** der vordere Überlappungsbereich (36) von Schrittabschnitt (8) zum Bauchabschnitt (4) wenigstens 12%, insbesondere 15 - 40 %, weiter insbesondere 15 - 35 % und weiter insbesondere 15 - 25 % der Fläche des Bauchabschnitts (4) einnimmt und der hintere Überlappungsbereich (38) von Schrittabschnitt (8) zum Rückenabschnitt (6) wenigstens 20%, insbesondere 20 - 40 %, weiter insbesondere 20-35 % und weiter insbesondere 22-32 % der Fläche des Rückenabschnitts (6) einnimmt.

10. Inkontinenzartikel nach einem oder mehreren der vorstehenden Ansprüche, **dadurch gekennzeichnet, dass** der Überhang (66a, 66b) des Backsheet-Materials (62) und/oder des Deckblatt-Materials (84) in Querrichtung (16) in der Summe, also beidseits der Längsränder (46) des Absorptionskörpers (7) wenigstens 25%, insbesondere 25 - 50 %, weiter insbesondere 30 - 45 % und weiter insbesondere 35 - 45 % bezogen auf die größte Breite (E) des Schrittabschnitts (8) beträgt.

11. Inkontinenzartikel nach einem oder mehreren der vorstehenden Ansprüche, **dadurch gekennzeichnet, dass** in dem zweiten beinöffnungsnahen Bauchteilabschnitt (22) und/oder in dem zweiten beinöffnungsnahen Rückenteilabschnitt (26) zweite Elastifizierungsmittel (40, 42) vorgesehen sind.

12. Inkontinenzartikel nach Anspruch 11, **dadurch gekennzeichnet, dass** sich die zweiten Elastifizierungsmittel (40, 42) ausgehend von den beiden Seitennahtbereichen (14) in Richtung (56) auf eine Längsmittelachse (44) des Inkontinenzartikels (2) erstrecken und dabei bogenförmig sich auffächernd mit zunehmendem Abstand voneinander verlaufen.

13. Inkontinenzartikel nach Anspruch 11 oder 12, **dadurch gekennzeichnet, dass** der zweite beinöffnungsnahe Bauchteilabschnitt (22) und der zweite beinöffnungsnahe Rückenteilabschnitt (26) jeweils mindestens eine erste Lage (221, 261) und eine zweite Lage (222, 262) aufweisen, zwischen denen die zweiten Elastifizierungsmittel (40,42) festgelegt, insbesondere mittels auf die erste und/oder zweite Lage vollflächig aufgebrachter adhäsiver Beschichtung (43) festgelegt sind.

14. Verfahren zum Herstellen von Inkontinenzartikel (2) in Höschenform für die Aufnahme von Körperausscheidungen mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), wobei der Schrittabschnitt mit einem bauchseitigen Längsende (98) und einem rückenseitigen Längsende (100) sich in einer Längsrichtung (9) zwischen dem Bauchabschnitt (4) mit einem schrittzugewandten Querrand (58) und dem Rückenabschnitt (6) mit einem schrittzugewandten Querrand (60) erstreckt, und wobei sich das bauchseitige Längsende (98) in Längsrichtung (9) bis zu einer parallel zur Querrichtung (16) verlaufenden gedachten Line L1 erstreckt, und wobei sich das rückenseitige Längsende (100) in Längsrichtung (9) bis zu einer parallel zur Querrichtung (16) verlaufenden gedachten Line L2 erstreckt, und wobei der Schrittabschnitt in einem vorderen Überlappungsbereich (36) den Bauchabschnitt (4) überlappt und an den Bauchabschnitt unlösbar angefügt ist und wobei der Schrittabschnitt in einem hinteren Überlappungsbereich (38) den Rückenabschnitt (6) überlappt und an den Rückenabschnitt (6) unlösbar angefügt ist, wobei der Schrittabschnitt (8) ein flüssigkeitsundurchlässiges Backsheet-Material (62) und ein Deckblatt-Material (84) umfasst, und wobei der Absorptionskörper (7) zwischen Backsheet-Material (62) und Deckblatt-Material (84) angeordnet ist, wobei das Deckblatt-Material (84) und/oder das Backsheet-Material (62) in Querrichtung (16) einen sich jeweils außerhalb von Längsrändern (46) des Absorptionskörpers (7) erstreckenden Überhang (66a, 66b) bilden, wobei sowohl der Schrittabschnitt (8) als auch der Bauchabschnitt (4) und der Rückenabschnitt (6) die Beinöffnungen (19) des Inkontinenzartikels begrenzen, wobei die Linie L1 den Bauchabschnitt (4) in einen ersten beinöffnungsfernen Bauchteilabschnitt (20) und einen zweiten beinöffnungsnahen Bauchteilabschnitt (22) und die Linie L2 den Rückenabschnitt (6) in einen ersten beinöffnungsfernen Rückenteilabschnitt (24) und einen zweiten beinöffnungsnahen Rückenteilabschnitt (26) teilt, wobei in dem ersten Bauchteilabschnitt (20) und dem ersten Rückenteilabschnitt (24) erste fadenförmige oder bandförmige Elastifizierungsmittel (28) mit einem Fadendurchmesser D vorgesehen sind, die sich in einem Abstand N voneinander und parallel zueinander in Quer- oder Hüftumfangsrichtung (16) erstrecken und so den ersten Bauchteilabschnitt (20) und den ersten Rückenteilabschnitt (24) flächenhaft elastifizieren, wobei der erste Bauchteilabschnitt und der erste Rückenteilabschnitt jeweils mindestens eine erste Lage (201, 241) und eine zweite Lage (202, 242) aufweisen, zwischen denen die ersten Elastifizierungsmittel (28) festgelegt sind, wobei das Festlegen der ersten Elastifzierungsmittel die folgenden Verfahrensschritte aufweist:

- Zuführen zweier die jeweils erste Lage (201, 241) des ersten Bauchteilabschnitts (20) und des ersten Rückenteilabschnitts (24) des Inkontinenzartikels (2) bildenden Teilbahnen (122, 124),
- Zuführen zweier die jeweils zweite Lage (202, 242) des ersten Bauchteilabschnitts (20) und des ersten Rückenabteilschnitts (24) bildenden Teilbahnen (134, 136),
- Aufbringen einer adhäsiven Beschichtung (33) in Form von streifenförmigen Kleberzonen (29) in Maschinenrichtung (120) mit einer Breite G parallel und in einem Abstand M zueinander auf mindestens eine der beiden den ersten Bauchteilabschnitt (20) bildenden Teilbahnen (122, 134) und auf mindestens eine der beiden den ersten Rückenteilabschnitt (24) bildenden Teilbahnen (124, 136),
- Zuführen von ersten Elastifizierungsmittel (28) mit einem Fadendurchmesser D in Maschinenrichtung (120) parallel und einem Abstand N zueinander, wobei der Abstand N der zugeführten benachbarten ersten Elastifizierungsmittel größer ist als der Abstand M benachbarter Kleberzonen (29) und Ablegen von jeweils einem einzigen ersten Elastifizierungsmittel (28) in Maschinenrichtung (120) innerhalb einer einzelnen Kleberzone (29) auf den den ersten Bauchteilabschnitt und auf den den ersten Rückenteilabschnitt bildenden Teilbahnen (122,124, 134, 136),
- Verbinden der ersten Elastifizierungsmittel (28) mit den Teilbahnen (122,124, 134, 136),
- Zuführen von Schrittabschnitten (8) und Zusammenführen der Schrittabschnitte (8) mit der den ersten Bauchteilabschnitt bildenden Teilbahnen (122, 134) und mit der den ersten Rückenabschnitt bildenden Teilbahnen (124, 136), derart, dass die Schrittabschnitte (8) in einer Längsrichtung (9) quer zur Maschinenrichtung (120) einenends mit der einen Teilbahn (122, 134) und anderenends mit der anderen Teilbahn (124, 136) im vorderen und hinteren Überlappungsbereich (36, 38) plan angeordnet sind und die Schrittabschnitte (8) mit einem Abstand in der Maschinenrichtung (120) voneinander angeordnet sind, und Fixieren der Schrittabschnitte (8) und Teilbahnen (122, 134, 124, 136) in den Überlappungsbereichen (36, 38),
- Falten um eine in Maschinenrichtung (120) verlaufende Faltlinie (150), derart, dass die eine Teilbahn (122,134) über die andere Teilbahn (124, 136) zu liegen kommt,
- Fügen der übereinander liegenden Teilbahnen (122, 134, 124, 136) quer zur Maschinenrichtung (120) in Abständen voneinander zur Ausbildung von Seitennahtbereichen (14) der herzustellenden Inkontinenzartikel (2), und Erhalt von einen Bauchabschnitt (4), einen Rückenabschnitt (6) und einen dazwischen angeordneten Schrittabschnitt (8) aufweisenden Produkten,
- Ausführen eines Trennschnitts quer zur Maschinenrichtung (120) und Erhalt von vereinzelten fertigen Inkontinenzartikeln (2).

**15.** Verfahren nach Anspruch 14, **dadurch gekennzeichnet, dass** auf jeweils beiden Teilbahnen, welche die erste und die zweite Lage des ersten Bauchteilabschnitts und die erste und zweite Lage des ersten Rückenteilabschnitts bilden, eine adhäsive Beschichtung (33) in Form von parallel zueinander angeordneten Kleberzonen (29) aufgebracht werden, wobei beide Teilbahnen mit insbesondere deckungsgleichen Auftragsmustern der adhäsiven Beschichtung versehen werden.

**16.** Verfahren nach Anspruch 14 oder 15, mit den weiteren Verfahrensschritten

- Zuführen und Aufbringen von zweiten Elastifizierungsmitteln (40, 42) auf den vorgegebenen den ersten Bauchteilabschnitt und/oder den ersten Rückenteilabschnitt bildenden Teilbahnen (122, 134, 124, 136) in einem von ersten Elastifizierungsmittel (28) freien Bereich oder auf jeweils einer weiteren zugeführten den Bauchabschnitt (4) und/oder den Rückenabschnitt (6) bildenden Teilbahnen (222, 262).

**17.** Verfahren nach Anspruch 16, **dadurch gekennzeichnet, dass** die zweiten Elastifizierungsmittel (40, 42) derart zugeführt werden, so dass sie sich ausgehend von den beiden Seitennahtbereichen (14) in Richtung (56) auf eine Längsmittelachse (44) des Inkontinenzartikels (2) erstrecken und dabei bogenförmig sich auffächernd mit zunehmendem Abstand voneinander verlaufen.

**Claims**

**1.** Incontinence article (2) in the form of pants for receiving bodily excretions, with a front stomach portion (4) and a rear back portion (6) which, in order to form a stomach and back band that is continuous in the transverse or waist-encircling direction (16) and has a waist opening (18) closed in the waist-encircling direction, are connected to each other by the manufacturer at side seam regions (14) on both sides, and with a crotch portion (8) which has an absorbent body (7), wherein the crotch portion, having a stomach-side long end (98) and a back-side long end (100), extends in a longitudinal direction (9) between the stomach portion (4) with a crotch-facing transverse periphery (58) and the back portion (6) with a crotch-facing transverse periphery (60), and wherein the stomach-side long end (98) extends in the longitudinal direction (9) as far as an imaginary line L1 running parallel to the transverse direction (16), and wherein the back-side long end (100) extends in the longitudinal direction (9) as far as an imaginary line L2 running parallel to the transverse direction (16), and wherein the crotch portion (8) overlaps the stomach portion (4) in a front overlapping region (36) and is joined inseparably to the stomach portion, and wherein the crotch portion (8) overlaps the back portion (6) in a rear overlapping region (38) and is joined inseparably to the back portion (6), wherein the crotch portion (8) comprises a liquid-impermeable backsheet material (62) and a topsheet material (84), and wherein the absorbent body (7) is arranged between backsheet material (62) and topsheet material (84), wherein the topsheet material (84) and/or the backsheet material (62) form, in transverse direction (16), an overhang (66a, 66b) that extends outside longitudinal peripheries (46) of the absorbent body (7), wherein not only the crotch portion (8) but also the stomach portion (4) and the back portion (6) delimit the leg openings (19) of the incontinence article, wherein the line L1 divides the stomach portion (4) into a first stomach subregion (20) away from the leg openings and a second stomach subregion (22) near the leg openings, and the line L2 divides the back portion (6) into a first back subregion (24) away from the leg openings and a second back subregion (26) near the leg openings, wherein first thread-like or band-like elasticating means (28) with a thread diameter D are provided in the first stomach subregion (20) and in the first back subregion (24) and extend at a distance N from one another, and parallel to one another, in the transverse or waist-encircling direction (16) and thus elasticate the first stomach subregion (20) and the first back subregion (24) across the surface areas thereof, wherein the first stomach subregion (20) and the first back subregion (24) each have at least a first layer (201, 241) and a second layer (202, 242) between which the first elasticating means (28) are secured by strip-shaped adhesive zones (29), wherein the strip-shaped adhesive zones, running parallel in the transverse direction or waist-encircling direction (16) and with a width G, are arranged at a distance M from one another, wherein the width G of the strip-shaped adhesive zones (29) is greater than the thread diameter D of the first elasticating means (28), and wherein a single first elasticating means (28) is in each case arranged within one respective strip-shaped adhesive zone (29).

**2.** Incontinence article according to Claim 1, **characterized in that** the quotient G/M is at least 0.10, in particular at least 0.30, more particularly at least 0.50, more particularly at least 0.75, more particularly at least 1.0, preferably at most 7.5, more preferably at most 6.0, more preferably at most 5.0, more preferably at most 4.0.

**3.** Incontinence article according to Claim 1 or 2, **characterized in that** the quotient G/D is at least 2, more preferably at least 4, more preferably at least 6, more preferably at least 8, more preferably at least 10, but preferably at most 100, more preferably at most 80, more preferably at most 60, more preferably at most 50, more preferably at most 40, more preferably at most 30.

4. Incontinence article according to one or more of the preceding claims, **characterized in that** the strip-shaped adhesive zones (29) comprise adhesive zone portions (29') having a length S and arranged at a distance P in the transverse direction (16), which adhesive zone portions (29') are preferably arranged in such a way that the quotient S/P is at least 0.1, more preferably at least 0.3, more preferably at least 0.5, more preferably at least 1.0, more preferably at least 2.0, but more preferably at most 15, more preferably at most 13, more preferably at most 10.

5. Incontinence article according to one or more of the preceding claims, **characterized in that** the first stomach subregion (22) away from the leg openings and/or the first back subregion (24) away from the leg openings occupies a proportion of 30-75%, more particularly of 35-70%, more particularly of 35-65%, relative to the surface area of the stomach portion (4) and/or of the back portion (6).

6. Incontinence article according to one or more of the preceding claims, **characterized in that** the first stomach subregion (22) away from the leg openings and/or the first back subregion (24) away from the leg openings has a surface (31), free from adhesive zones (29) and/or adhesive zone portions (29'), with a proportion of 20-80%, in particular of 25-70%, more particularly of 30-60%, relative to the surface area of the first stomach subregion (22) away from the leg openings and/or of the first back subregion (24) away from the leg openings.

7. Incontinence article according to one or more of the preceding claims, **characterized in that** the adhesive zones and/or adhesive zone portions (29, 29') have an adhesive coating (33) with an area density of 2-40 g/m$^2$, more particularly 2-30 g/m$^2$, more particularly 2-20 g/m$^2$, more particularly 2-10 g/m$^2$.

8. Incontinence article according to one or more of the preceding claims, **characterized in that** the adhesive coating (33) is applied to the first and/or second layer of the first stomach subregion (22) and to the first and/or second layer of the first back subregion (24).

9. Incontinence article according to one or more of the preceding claims, **characterized in that** the front overlapping region (36) of crotch portion (8) to stomach portion (4) occupies at least 12%, in particular 15-40%, more particularly 15-35% and more particularly 15-25% of the surface of the stomach portion (4), and the rear overlapping region (38) from crotch portion (8) to back portion (6) occupies at least 20%, in particular 20-40%, more particularly 20-35% and more particularly 22-32% of the surface of the back portion (6).

10. Incontinence article according to one or more of the preceding claims, **characterized in that** the overhang (66a, 66b) of the backsheet material (62) and/or of the topsheet material (84) in the transverse direction (16) is in total, that is to say on both sides of the longitudinal peripheries (46) of the absorbent body (7), at least 25%, in particular 25-50%, more particularly 30-45% and more particularly 35-45%, relative to the greatest width (E) of the crotch portion (8).

11. Incontinence article according to one or more of the preceding claims, **characterized in that** second elasticating means (40, 42) are provided in the second stomach subregion (22) near the leg openings and/or in the second back subregion (26) near the leg openings.

12. Incontinence article according to Claim 11, **characterized in that** the second elasticating means (40, 42) extend from the two side seam regions (14) in the direction (56) of a longitudinal centre axis (44) of the incontinence article (2) and thereby run in an arcuately fanning-out manner with increasing distance from one another.

13. Incontinence article according to Claim 11 or 12, **characterized in that** the second stomach subregion (22) near the leg openings and the second back subregion (26) near the leg openings each have at least a first layer (221, 261) and a second layer (222, 262), between which the second elasticating means (40, 42) are secured, in particular secured by means of adhesive coating (43) applied to the first and/or second layer over the whole surface thereof.

14. Method for producing an incontinence article (2) in the form of pants for receiving bodily excretions, with a front stomach portion (4) and a rear back portion (6) which, in order to form a stomach and back band that is continuous in the transverse or waist-encircling direction (16) and has a waist opening (18) closed in the waist-encircling direction, are connected to each other by the manufacturer at side seam regions (14) on both sides, and with a crotch portion (8) which has an absorbent body (7), wherein the crotch portion, having a stomach-side long end (98) and a back-side long end (100), extends in a longitudinal direction (9) between the stomach portion (4) with a crotch-facing transverse periphery (58) and the back portion (6) with a crotch-facing transverse periphery (60), and wherein the stomach-side long end (98) extends in the longitudinal direction (9) as far as an imaginary line L1 running parallel to the transverse direction (16), and wherein the back-side long end (100) extends in the longitudinal direction (9) as far as an imaginary line L2 running parallel to the transverse direction (16), and wherein the crotch portion overlaps the stomach portion (4) in a front overlapping region (36) and is joined inseparably to the stomach portion, and wherein the crotch portion overlaps the back portion (6) in a rear overlapping region (38) and is joined inseparably to the back portion (6), wherein the crotch portion (8) comprises a liquid-impermeable backsheet material (62) and a topsheet material (84), and wherein the absorbent body (7) is arranged between backsheet material (62) and topsheet material (84), wherein the topsheet material (84) and/or the backsheet material (62) form, in transverse direction (16), an overhang (66a, 66b) that extends outside longitudinal peripheries (46) of the absorbent body (7), wherein not only the crotch portion (8) but also the stomach portion (4) and the back portion (6) delimit the leg openings (19) of the incontinence article, wherein the line L1 divides the stomach portion (4) into a first stomach subregion (20) away from the leg openings and a second stomach subregion (22) near the leg openings, and the line L2 divides the back portion (6) into a first back subregion (24) away from the leg openings and a second back subregion (26) near the leg openings, wherein first thread-like or band-like elasticating means (28) with a thread diameter D are provided in the first stomach subregion (20) and in the first back subregion (24) and extend at a distance N from one another, and parallel to one another, in the transverse or waist-encircling direction (16) and thus elasticate the first stomach subregion (20) and the first back subregion (24) across the surface areas thereof, wherein the first stomach subregion and the first back subregion each have at least a first layer (201, 241) and a second layer (202, 242) between which the first elasticating means (28) are secured, wherein securing of the first elasticating means has the following method steps:

- supplying two subsidiary webs (122, 124) forming the respective first layer (201, 241) of the first stomach subregion (20) and of the first back subregion (24) of the incontinence article (2),
- supplying two subsidiary webs (134, 136) forming the respective second layer (202, 242) of the first stomach subregion (20) and of the first back subregion (24),
- applying an adhesive coating (33), in the form of strip-shaped adhesive zones (29) parallel in a machine direction (120) and with a width G and at a distance M from each other, to at least one of the two subsidiary webs (122, 134) forming the first stomach subregion (20) and to at least one of the two subsidiary webs (124, 136) forming the first back subregion (24),
- supplying first elasticating means (28) with a thread diameter D in a machine direction (120), parallel and at a distance N from one another, wherein the distance N of the supplied adjacent first elasticating means is greater than the distance M of adjacent adhesive zones (29), and placing in each case a single first elasticating means (28) in machine direction (120) within an individual adhesive zone (29) onto the subsidiary webs (122, 124, 134, 136) forming the first stomach subregion and forming the first back subregion,
- connecting the first elasticating means (28) to the subsidiary webs (122, 124, 134, 136),
- supplying crotch portions (8) and bringing the crotch portions (8) together with the subsidiary webs (122, 134) forming the first stomach subregion and with the subsidiary webs (124, 136) forming the first back portion in such a way that the crotch portions (8), in a longitudinal direction (9) transverse to the machine direction (120), are arranged in plane at one end with one subsidiary web (122, 134) and at the other end with the other subsidiary web (124, 136) in the front and rear overlapping region (36, 38), and the crotch portions (8) are arranged at a distance from one another in the machine direction (120), and fixing the crotch portions (8) and subsidiary webs (122, 134, 124, 136) in the overlapping regions (36, 38),
- folding about a fold line (150) that runs in the machine direction (120), in such a way that one subsidiary web (122, 134) comes to lie over the other subsidiary web (124, 136),
- joining the superposed subsidiary webs (122, 134, 124, 136) transversely with respect to the machine direction (120) at distances from one another in order to form side seam regions (14) of the incontinence article (2) to be produced, and obtaining products that have a stomach portion (4), a back portion (6) and, arranged between these, a crotch portion (8),
- making a separating cut transversely with respect to the machine direction (120) and obtaining individual, finished incontinence articles (2).

15. Method according to Claim 14, **characterized in that** an adhesive coating (33) in the form of mutually parallel adhesive zones (29) is applied in each case to the two subsidiary webs that form the first and second layers of the first stomach subregion and the first and second layers of the first back subregion, wherein both subsidiary webs are provided in particular with congruent application patterns of the adhesive coating.

**16.** Method according to Claim 14 or 15, with the further method steps of

- supplying and applying second elasticating means (40, 42) on the predefined subsidiary webs (122, 134, 124, 136) forming the first stomach subregion and/or the first back subregion, in an area free of first elasticating means (28), or on a further supplied subsidiary web (222, 262) forming the stomach portion (4) and/or the back portion (6).

**17.** Method according to Claim 16, **characterized in that** the second elasticating means (40, 42) are supplied in such a way that they extend from the two side seam regions (14) in the direction (56) of a longitudinal centre axis (44) of the incontinence article (2) and thereby run in an arcuately fanning-out manner with increasing distance from one another.

**Revendications**

**1.** Article pour incontinence (2) en forme de culotte pour la réception de pertes corporelles avec une partie ventrale antérieure (4) et une partie dorsale postérieure (6), qui sont reliées l'une à l'autre chez le fabricant à des régions (14) de couture latérales sur les deux côtés pour la formation d'une bande ventrale et dorsale continue dans la direction transversale ou du pourtour des hanches (16) avec une ouverture de hanche (18) fermée dans la direction du pourtour des hanches, et avec une partie d'entrejambe (8) présentant un corps absorbant (7), dans lequel la partie d'entrejambe s'étend avec une extrémité longitudinale côté ventral (98) et une extrémité longitudinale côté dorsal (100) dans une direction longitudinale (9) entre la partie ventrale (4) avec un bord transversal (58) tourné vers l'entrejambe et la partie dorsale (6) avec un bord transversal (60) tourné vers l'entrejambe, et dans lequel l'extrémité longitudinale côté ventral (98) s'étend en direction longitudinale (9) jusqu'à une ligne imaginaire L1 s'étendant parallèlement à la direction transversale (16), et dans lequel l'extrémité longitudinale côté dorsal (100) s'étend en direction longitudinale (9) jusqu'à une ligne imaginaire L2 s'étendant parallèlement à la direction transversale (16), et dans lequel la partie d'entrejambe (8) recouvre la partie ventrale (4) dans une zone de recouvrement antérieure (36) et est attachée de façon inséparable à la partie ventrale et dans lequel la partie d'entrejambe (8) recouvre la partie dorsale (6) dans une zone de recouvrement postérieure (38) et est attachée de façon inséparable à la partie dorsale (6), dans lequel la partie d'entrejambe (8) comprend un matériau de feuille de support imperméable au liquide (62) et un matériau de feuille de recouvrement (84), et dans lequel le corps absorbant (7) est disposé entre le matériau de feuille de support (62) et le matériau de feuille de recouvrement (84), dans lequel le matériau de feuille de recouvrement (84) et/ou le matériau de feuille de support (62) forment en direction transversale (16) un débordement (66a, 66b) s'étendant respectivement à l'extérieur de bords longitudinaux (46) du corps absorbant (7), dans lequel aussi bien la partie d'entrejambe (8) que la partie ventrale (4) et la partie dorsale (6) limitent les ouvertures de jambe (19) de l'article pour incontinence, dans lequel la ligne L1 divise la partie ventrale (4) en une première partie ventrale partielle (20) éloignée des ouvertures de jambe et une deuxième partie ventrale partielle (22) proche des ouvertures de jambe et la ligne L2 divise la partie dorsale (6) en une première partie dorsale partielle (24) éloignée des ouvertures de jambe et une deuxième partie dorsale partielle (26) proche des ouvertures de jambe, dans lequel il est prévu dans la première partie ventrale partielle (20) et dans la première partie dorsale partielle (24) des premiers moyens d'élastification (28) en forme de fil ou en forme de bande avec un diamètre de fil D, qui s'étendent à une première distance N l'un de l'autre et parallèlement l'un à l'autre dans la direction transversale ou du pourtour des hanches (16), qui rendent ainsi élastiques à plat la première partie ventrale partielle (20) et la première partie dorsale partielle (24), dans lequel la première partie ventrale partielle (20) et la première partie dorsale partielle (24) présentent respectivement au moins une première couche (201, 241) et une deuxième couche (202, 242), entre lesquelles les premiers moyens d'élastification (28) sont fixés au moyen de zones adhésives en forme de rubans (29), dans lequel les zones adhésives en forme de rubans sont disposées dans la direction transversale ou du pourtour des hanches (16) avec une largeur G parallèlement et à une distance M l'une de l'autre, dans lequel la largeur G des zones adhésives en forme de rubans (29) est plus grande que le diamètre de fil D des premiers moyens d'élastification (28), et dans lequel un unique premier moyen d'élastification (28) est chaque fois disposé à l'intérieur d'une zone adhésive en forme de ruban respective (29).

**2.** Article pour incontinence selon la revendication 1, **caractérisé en ce que** le quotient G/M vaut au moins 0,10, en particulier au moins 0,30, en particulier encore au moins 0,50, en particulier encore au moins 0,75, en particulier encore au moins 1,0, de préférence au plus 7,5, de préférence encore au plus 6,0, de préférence encore au plus 5,0, de préférence encore au plus 4,0.

**3.** Article pour incontinence selon la revendication 1 ou 2, **caractérisé en ce que** le quotient G/D vaut au moins 2, de préférence au moins 4, de préférence encore au moins 6, de préférence encore au moins 8, de préférence encore au moins 10, mais de préférence au plus 100, de préférence encore au plus 80, de préférence encore au plus 60, de préférence encore au plus 50, de préférence encore au plus 40, et de préférence encore au plus 30.

4. Article pour incontinence selon une ou plusieurs des revendications précédentes, **caractérisé en ce que** les zones adhésives en forme de rubans (29) comprennent des parties de zones adhésives (29') de longueur S disposées à une distance P dans la direction transversale (16), qui sont de préférence disposées de telle manière que le quotient S/P vaille au moins 0,1, de préférence au moins 0,3, de préférence encore au moins 0,5, de préférence encore au moins 1,0, de préférence encore au moins 2,0, mais de préférence au plus 15, de préférence encore au plus 13, de préférence encore au plus 10.

5. Article pour incontinence selon une ou plusieurs des revendications précédentes, **caractérisé en ce que** la première partie ventrale partielle (22) éloignée des ouvertures de jambe et/ou la première partie dorsale partielle (24) éloignée des ouvertures de jambe occupe une proportion de 30 - 75 %, en particulier de 35 - 70 %, en particulier encore de 35 - 65 % par rapport à la surface de la partie ventrale (4) et/ou de la partie dorsale (6).

6. Article pour incontinence selon une ou plusieurs des revendications précédentes, **caractérisé en ce que** la première partie ventrale partielle (22) éloignée des ouvertures de jambe et/ou la première partie dorsale partielle (24) éloignée des ouvertures de jambe présente une surface (31) libre de zones adhésives (29) et/ou de parties de zones adhésives (29') avec une proportion de 20 - 80 %, en particulier de 25 - 70 %, en particulier encore de 30 - 60 % par rapport à la surface de la première partie ventrale partielle (22) éloignée des ouvertures de jambe et/ou de la première partie dorsale partielle (24) éloignée des ouvertures de jambe.

7. Article pour incontinence selon une ou plusieurs des revendications précédentes, **caractérisé en ce que** les zones adhésives et/ou les parties de zones adhésives (29, 29') présentent un revêtement adhésif (33) avec un grammage de 2 - 40 g/m$^2$, en particulier de 2 - 30 g/m$^2$, en particulier encore de 2 - 20 g/m$^2$, et en particulier encore de 2 - 10 g/m$^2$.

8. Article pour incontinence selon une ou plusieurs des revendications précédentes, **caractérisé en ce que** le revêtement adhésif (33) est déposé sur la première et/ou la deuxième couche de la première partie ventrale partielle (22) et sur la première et/ou la deuxième couche de la première partie dorsale partielle (24).

9. Article pour incontinence selon une ou plusieurs des revendications précédentes, **caractérisé en ce que** la zone de recouvrement antérieure (36) de la partie d'entrejambe (8) sur la partie ventrale (4) occupe au moins 12 %, en particulier 15 - 40 %, en particulier encore 15 - 35 % et en particulier encore 15 - 25 % de la surface de la partie ventrale (4) et la zone de recouvrement postérieure (38) de la partie d'entrejambe (8) sur la partie dorsale (6) occupe au moins 20 %, en particulier 20 - 40 %, en particulier encore 20 - 35 % et en particulier encore 22 - 32 % de la surface de la partie dorsale (6).

10. Article pour incontinence selon une ou plusieurs des revendications précédentes, **caractérisé en ce que** le débordement (66a, 66b) du matériau de la feuille de support (62) et/ou du matériau de la feuille de recouvrement (84) vaut, en sommation en direction transversale (16), donc de part et d'autre des bords longitudinaux (46) du corps absorbant (7), au moins 25 %, en particulier 25 - 50 %, en particulier encore 30 - 45 % et en particulier encore 35 - 45 % par rapport à la plus grande largeur (E) de la partie d'entrejambe (8).

11. Article pour incontinence selon une ou plusieurs des revendications précédentes, **caractérisé en ce qu'**il est prévu des deuxièmes moyens d'élastification (40, 42) dans la deuxième partie ventrale partielle (22) proche des ouvertures de jambe et/ou dans la deuxième partie dorsale partielle (26) proche des ouvertures de jambe.

12. Article pour incontinence selon la revendication 11, **caractérisé en ce que** les deuxièmes moyens d'élastification (40, 42) s'étendent à partir des deux zones de couture latérales (14) en direction (56) d'un axe central longitudinal (44) de l'article pour incontinence (2) et s'étendent en l'occurrence en forme d'arc en s'échelonnant à distance croissante l'un de l'autre.

13. Article pour incontinence selon la revendication 11 ou 12, **caractérisé en ce que** la deuxième partie ventrale partielle (22) proche des ouvertures de jambe et la deuxième partie dorsale partielle (26) proche de l'ouverture de jambes présentent respectivement au moins une première couche (221, 261) et une deuxième couche (222, 262), entre lesquelles les deuxièmes moyens d'élastification (40, 42) sont fixés, sont en particulier fixés au moyen d'un revêtement adhésif (43) déposé en pleine surface sur la première et/ou la deuxième couche.

**14.** Procédé de fabrication d'un article pour incontinence (2) en forme de culotte pour la réception de pertes corporelles avec une partie ventrale antérieure (4) et une partie dorsale postérieure (6), qui sont reliées l'une à l'autre chez le fabricant à des régions de couture latérales (14) sur les deux côtés pour la formation d'une bande ventrale et dorsale continue dans la direction transversale ou du pourtour des hanches (16) avec une ouverture de hanche (18) fermée dans la direction du pourtour des hanches, et avec une partie d'entrejambe (8) présentant un corps absorbant (7), dans lequel la partie d'entrejambe s'étend avec une extrémité longitudinale côté ventral (98) et une extrémité longitudinale côté dorsal (100) dans une direction longitudinale (9) entre la partie ventrale (4) avec un bord transversal (58) tourné vers l'entrejambe et la partie dorsale (6) avec un bord transversal (60) tourné vers l'entrejambe, et dans lequel l'extrémité longitudinale côté ventral (98) s'étend en direction longitudinale (9) jusqu'à une ligne imaginaire L1 s'étendant parallèlement à la direction transversale (16), et dans lequel l'extrémité longitudinale côté dorsal (100) s'étend en direction longitudinale (9) jusqu'à une ligne imaginaire L2 s'étendant parallèlement à la direction transversale (16), et dans lequel la partie d'entrejambe (8) recouvre la partie ventrale (4) dans une zone de recouvrement antérieure (36) et est attachée de façon inséparable à la partie ventrale et dans lequel la partie d'entrejambe (8) recouvre la partie dorsale (6) dans une zone de recouvrement postérieure (38) et est attachée de façon inséparable à la partie dorsale (6), dans lequel la partie d'entrejambe (8) comprend un matériau de feuille de support imperméable au liquide (62) et un matériau de feuille de recouvrement (84), et dans lequel le corps absorbant (7) est disposé entre le matériau de feuille de support (62) et le matériau de feuille de recouvrement (84), dans lequel le matériau de feuille de recouvrement (84) et/ou le matériau de feuille de support (62) forment en direction transversale (16) un débordement (66a, 66b) s'étendant respectivement à l'extérieur de bords longitudinaux (46) du corps absorbant (7), dans lequel aussi bien la partie d'entrejambe (8) que la partie ventrale (4) et la partie dorsale (6) limitent les ouvertures de jambe (19) de l'article pour incontinence, dans lequel la ligne L1 divise la partie ventrale (4) en une première partie ventrale partielle (20) éloignée des ouvertures de jambe et une deuxième partie ventrale partielle (22) proche des ouvertures de jambe et la ligne L2 divise la partie dorsale (6) en une première partie dorsale partielle (24) éloignée des ouvertures de jambe et une deuxième partie dorsale partielle (26) proche des ouvertures de jambe, dans lequel il est prévu dans la première partie ventrale partielle (20) et dans la première partie dorsale partielle (24) des premiers moyens d'élastification (28) en forme de fil ou en forme de bande avec un diamètre de fil D, qui s'étendent à une première distance N l'un de l'autre et parallèlement l'un à l'autre dans la direction transversale ou du pourtour des hanches (16), qui rendent ainsi élastiques à plat la première partie ventrale partielle (20) et la première partie dorsale partielle (24), dans lequel la première partie ventrale partielle et la première partie dorsale partielle présentent respectivement au moins une première couche (201, 241) et une deuxième couche (202, 242) entre lesquelles les premiers moyens d'élastification (28) sont fixés, dans lequel la fixation des premiers moyens d'élastification présente les étapes suivantes:

- fourniture de deux bandes partielles (122, 124) formant respectivement la première couche (201, 241) de la première partie ventrale partielle (20) et de la première partie dorsale partielle (24) de l'article pour incontinence (2),
- fourniture de deux bandes partielles (134, 136) formant respectivement la deuxième couche (202, 242) de la première partie ventrale partielle (20) et de la première partie dorsale partielle (24),
- application d'un revêtement adhésif (33) sous la forme de zones adhésives en forme de rubans (29) dans le sens machine (120) avec une largeur G parallèlement et à une distance M l'une de l'autre sur au moins une des deux bandes partielles (122, 134) formant la première partie ventrale partielle (20) et sur au moins une des deux bandes partielles (124, 136) formant la première partie dorsale partielle (24),
- fourniture de premiers moyens d'élastification (28) avec un diamètre de fil D dans le sens machine (120) parallèlement et à une distance N l'un de l'autre, dans lequel la distance N des premiers moyens d'élastification voisins fournis est plus grande que la distance M de zones adhésives voisines (29) et pose respectivement d'un seul premier moyen d'élastification (28) dans le sens machine (120) à l'intérieur d'une seule zone adhésive (29) sur les bandes partielles (122, 134) formant la première partie ventrale partielle et sur les bandes partielles (124, 136) formant la première partie dorsale partielle,
- liaison des premiers moyens d'élastification (28) aux bandes partielles (122, 124, 134, 136),
- fourniture de parties d'entrejambe (8) et assemblage des parties d'entrejambe (8) avec les bandes partielles (122, 134) formant la première partie ventrale partielle et avec les bandes partielles (124, 136) formant la première partie dorsale partielle, de telle manière que les parties d'entrejambe (8) soient disposées de façon plane, dans une direction longitudinale (9) transversale au sens machine (120), à une extrémité avec une première bande partielle (122, 134) et à l'autre extrémité avec l'autre bande partielle (124, 136) dans la zone de recouvrement antérieure et postérieure (36, 38) et que les parties d'entrejambe (8) soient disposées avec une distance l'une de l'autre dans le sens machine (120), et fixation des parties d'entrejambe (8) et des bandes partielles (122, 124, 134, 136) dans les zones de recouvrement (36, 38),
- pliage autour d'une ligne de pliage (150) s'étendant dans le sens machine (120), de telle manière qu'une première bande partielle (122, 134) vienne se placer au-dessus de l'autre bande partielle (124, 136),
- liaison des bandes partielles superposées (122, 134, 124, 136) transversalement au sens machine (120) à des distances l'une de l'autre pour former des zones de couture latérales (14) de l'article pour incontinence à fabriquer (2), et obtention de produits présentant une partie ventrale (4), une partie dorsale (6) et une partie d'entrejambe (8) disposée entre celles-ci,
- exécution d'une coupe transversalement au sens  machine (120) et obtention d'articles pour incontinence (2) individuels terminés.

**15.** Procédé selon la revendication 14, **caractérisé en ce que** l'on applique sur respectivement les deux bandes partielles, qui forment la première et la deuxième couches de la première partie ventrale partielle et la première et la deuxième couches de la première partie dorsale partielle, un revêtement adhésif (33) sous la forme de zones adhésives (29) disposées parallèlement les unes aux autres, dans lequel les deux bandes partielles sont dotées de motifs déposés du revêtement adhésif qui se recouvrent en particulier exactement.

**16.** Procédé selon la revendication 14 ou 15, comportant les étapes supplémentaires suivantes:

- fournir et appliquer des deuxièmes moyens d'élastification (40, 42) sur les bandes partielles prédéterminées (122, 134, 124, 136) formant la première partie ventrale partielle et/ou la première partie dorsale partielle, dans une zone libre de premiers moyens d'élastification (28) ou sur respectivement une autre bande partielle fournie (222, 262) formant la partie ventrale (4) et/ou la partie dorsale (6).

**17.** Procédé selon la revendication 16, **caractérisé en ce que** l'on fournit les deuxièmes moyens d'élastification (40, 42) de telle manière qu'ils s'étendent à partir des deux zones de couture latérales (14) en direction (56) d'un axe central longitudinal (44) de l'article pour incontinence (2) et qu'ils s'étendent en l'occurrence en forme d'arc en s'échelonnant à distance croissante l'un de l'autre.

*Fig. 1*

*Fig. 2*

*Fig. 3*

16

48  62  46  84  7  8  46  48

66a  66b

6  E

*Fig. 4*

9

201  28  L1  98 88  84 8  7  89  L2  241

67a  62  67b

20  22  4  6  26  24

202  29,33  242

*Fig. 5*

*Fig. 6*

Fig. 7

Fig. 8

Fig. 9

Fig. 10

Fig. 11a

Fig. 11b

Fig. 11c

Fig. 12

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004052260 A1
- WO 03039423 A1
- WO 2009065499 A1
- EP 1938777 A2
- WO 2003039422 A1
- EP 1830770 A1
- WO 2009080180 A1